# DEMANDE DE BREVET EUROPEEN

(11) **EP 0 926 145 A1**
(43) Date de publication de la demande: **30.06.1999**
(21) Numéro de dépôt: 98403272.2
(22) Date de dépôt: 23.12.1998
(51) Int. Cl.: C07D 307/81, A61K 31/34, A61K 31/38, A61K 31/44, C07D 333/58, C07D 333/60, C07D 405/06, C07D 409/06, C07D 493/04, C07D 407/04, C07D 495/04

(54) **Composés naphtaléniques, leur procédé de préparation et les compositions pharmaceutiques qui les contiennent**

(30) Priorité: 24.12.1997 FR 9716468
(71) Demandeur: ADIR ET COMPAGNIE, 92415 Courbevoie Cédex (FR)
(72) Inventeur: Lesieur, Daniel, 59147 Gondecourt (FR); Ruiz, Nicolas, 75017 Paris (FR); Wallez, Valérie, 59117 Wervicq-Sud (FR); Boye, Sophie, 94300 Vincennes (FR); Bennejean, Caroline, 94220 Charenton le Pont (FR); Renard, Pierre, 78150 Le Chesnay (FR); Delagrange, Philippe, 92130 Issy les Moulineaux (FR)

(57) **Abrégé**

Composé de formule (I) : 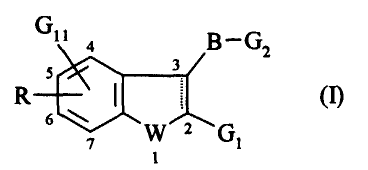 dans laquelle :
- B représente une chaîne alkylène (C₁-C₆) éventuellement substituée,
- W représente un atome d'oxygène ou de soufre,
- R représente un atome d'hydrogène, un groupement hydroxy, R' ou OR', R' étant défini dans la description,
- G₁ et G₁₁ sont tels que définis dans la description,
- G₂ représente un groupement choisi parmi : 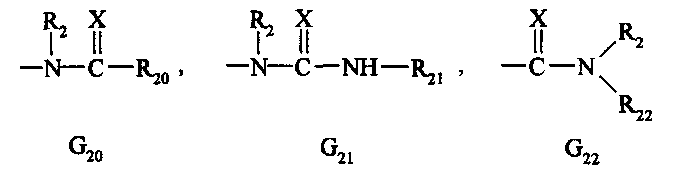 dans lesquels R₂₀, R₂₁, R₂₂, et R₂ sont tels que définis dans la description.
Médicaments

## Description

La présente invention concerne de nouveaux composés hétérocycliques, leur procédé de préparation, et les compositions pharmaceutiques qui les contiennent.

Les demandes de brevets EP 721938 et EP 721947 décrivent des dérivés hétérocycliques de type arylalkylamide et arylalkylurée, ligands des récepteurs de la mélatonine. Ces composés se caractérisent par la présence d'un seul substituant sur le noyau portant la chaîne alkylamide. Dans le brevet EP 527687 des composés de structure voisine ont été étudiés pour leur action au niveau du système mélatoninergique. Ces dérivés se caractérisent par la présence d'une chaîne N-cycloalkylamidoalkyle greffée sur un noyau benzo[*b*]thiophène et benzo[*b*]furane. De tels hétérocycles sont également décrits dans les demandes EP 745583 et WO 9706140 en tant que ligands mélatoninergiques et se caractérisent par la présence d'un substituant de type acyle ou alkyle en des positions choisies du noyau hétérocyclique.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et sur le traitement de l'obésité (International Journal of Eating Disorders, 1996, 20 (4), pp 443-446).

Ces différents effets s'exercent par l'intermédiaire de récepteurs spécifiques de la mélatonine. Des études de biologie moléculaire ont montré l'existence de plusieurs sous-types de récepteurs pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention présentent une structure originale se caractérisant par un noyau hétérocyclique substitué par une chaîne alklylamide et présentant en plus deux substituants sur le noyau principal. Cette structure leur confère de façon surprenante une très grande affinité pour les récepteurs de la mélatonine et une sélectivité pour l'un ou l'autre des sous-types réceptoriels.

L'invention concerne les composés de formule (I) : 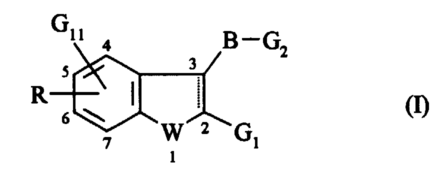 dans laquelle :
- la liaison 2-3 est saturée ou insaturée
- W représente un atome d'oxygène ou de soufre,
- B représente une chaîne alkylène (C₁-C₆) linéaire ou ramifiée éventuellement substituée par un ou plusieurs groupements alkyle(C₁-C₆) linéaire ou ramifié, hydroxy, alkoxy (C₁-C₆) linéaire ou ramifié, carboxy, et/ou alkoxycarbonyle (C₁-C₆) linéaire ou ramifié,
- R représente un atome d'hydrogène, un groupement hydroxy, un radical R' ou un groupement OR', R' représentant un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, trihalogénoalkylsulfonyl (C₁-C₆) linéaire ou ramifié, aryle éventuellement substitué, biphényle éventuellement substitué, ou hétéroaryle éventuellement substitué,
- G₁ représente un atome d'halogène, un groupement trihalogénoalkylsulfonyloxy (C₁-C₆) linéaire ou ramifié, carboxy, formyl, cyano, un radical R₁, un groupement -CO-R₁ ou -O-CO-R₁, R₁ représentant un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, aryle éventuellement substitué, biphényle éventuellement substitué, ou hétéroaryle éventuellement substitué,
   et dans ce cas G₁₁ représente un atome d'hydrogène ou G₁₁ et R forment ensemble avec les atomes de carbone qui les portent un cycle de 5 à 7 chaînons, saturé ou insaturé, contenant un atome d'oxygène, ce cycle étant éventuellement substitué par un ou plusieurs groupements choisis parmi alkyle (C₁-C₆) linéaire ou ramifié, alkoxy (C₁-C₆) linéaire ou ramifié, hydroxy, oxo, carboxy, ou alkoxycarbonyle (C₁-C₆) linéaire ou ramifié,
   ou bien,
   G₁ représente un atome d'hydrogène, et dans ce cas, G₁₁ représente un groupement trihalogénoalkylsulfonyloxy (C₁-C₆) linéaire ou ramifié, carboxy, formyl, cyano, un radical R₁, un groupement -CO-R₁ ou -O-CO-R₁, R₁ représentant un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, aryle éventuellement substitué, biphényle éventuellement substitué, ou hétéroaryle éventuellement substitué,
- G₂ représente un groupement choisi parmi : 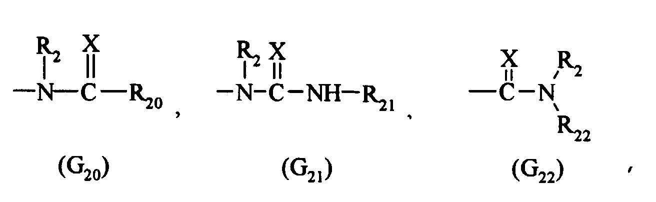 dans lesquels :
   - X représente un atome d'oxygène ou de soufre,
   - R₂ représente un atome d'hydrogène ou un groupement alkyle (C₁-C₆) linéaire ou ramifié,
   - R₂₀, R₂₁, R₂₂ représentent un groupement alkyle (C₁-C₆) linéaire ou ramifié éventuellement substitué, akényle (C₂-C₆) linéaire ou ramifié éventuellement substitué, alkynyle (C₂-C₆) linéaire ou ramifié éventuellement substitué, cycloalkyle (C₃-C₇) éventuellement substitué, hétéroaryle éventuellement substitué, aryle éventuellement substitué, ou biphényle éventuellement substitué,
   étant entendu que :
   - lorsque G₂ représente un groupement G₂₂ dans lequel R₂₂ tel que défini précédemment est différent d'un groupement aryle éventuellement substitué, hétéroaryle éventuellement substitué, ou biphényle éventuellement substitué, alors G₁ ou G₁₁ représentent un groupement hétéroaryle (différent de pyridyle) éventuellement substitué, un groupement alkyle (C₁-C₆) linéaire ou ramifié substitué par un groupement hétéroaryle (différent de pyridyle) éventuellement substitué, ou un groupement -CO-R₁ ou O-CO-R₁, R₁ étant tel que défini précédemment,
   - lorsque G₂ représente un groupement G₂₀ ou G₂₁ dans lesquels R₂₀ et R₂₁ sont différents d'un groupement aryle éventuellement substitué, biphényle éventuellement substitué, ou hétéroaryle éventuellement substitué, et G₁ est différent d'un groupement hétéroaryle éventuellement substitué, ou naphtyle éventuellement substitué, ou G₁₁ est différent d'un groupement hétéroaryle éventuellement substitué, ou naphtyle éventuellement substitué tandis que R représente un atome d'hydrogène, alors B représente une chaîne méthylène ou éthylène éventuellement substituée,
   - lorsque G₂ représente un groupement G₂₀ ou G₂₁, R₂₀ ou R₂₁ tels que définis précédemment étant différents d'un groupement aryle éventuellement substitué et d'un groupement hétéroaryle éventuellement substitué, et :
      ↪ lorsque R et G₁ représentent chacun un atome d'hydrogène, alors G₁₁ est différent d'un groupement alkyle (C₁-C₆) linéaire ou ramifié, d'un groupement cycloalkyle (C₃-C₇) éventuellement substitué par un ou plusieurs atomes d'halogène ou groupements hydroxy, et différent d'un groupement alkyle (C₁-C₆) linéaire ou ramifié substitué par un ou plusieurs atomes d'halogène ou groupements hydroxy, ou alkoxy (C₁-C₆) linéaire ou ramifié, ou cycloalkyle (C₃-C₇) éventuellement substitué par un ou plusieurs atomes d'halogène ou groupements hydroxy,
      ↪ lorsque G₁ représente un atome d'hydrogène et R représente un groupement OR', R' tel que défini précédemment étant différent d'un groupement hétéroaryle éventuellement substitué, alors G₁₁ est différent d'un groupement alkyle (C₁-C₆) linéaire ou ramifié, d'un groupement alkényle (C₂-C₆) linéaire ou ramifié, et d'un groupement alkynyle (C₂-C₆) linéaire ou ramifié,
   - lorsque G₂ représente soit un groupement G₂₁ dans lequel R₂₁ est différent d'un groupement alkényle (C₂-C₆) linéaire ou ramifié éventuellement substitué et d'un groupement alkynyle (C₂-C₆) éventuellement substitué, soit un groupement G₂₀ dans lequel R₂₀ représente un groupement cycloalkyle (C₃-C₇) éventuellement substitué par un ou plusieurs atomes d'halogène ou groupements hydroxy ou alkoxy (C₁-C₆) linéaire ou ramifié, ou un groupement alkyle (C₁-C₆) linéaire ou ramifié substitué par un groupement cycloalkyle (C₃-C₇) éventuellement substitué par un ou plusieurs atomes d'halogène ou groupements hydroxy ou alkoxy (C₁-C₆) linéaire ou ramifié, alors :
      ↪ G₁₁ est différent d'un groupement alkyle (C₁-C₆) linéaire ou ramifié,
      ↪ G₁ est différent d'un atome d'halogène, d'un groupement alkyl (C₁-C₆) linéaire ou ramifié, d'un groupement alkyle (C₁-C₆) linéaire ou ramifié substitué par un groupement phényle éventuellement substitué, et d'un groupement phényle éventuellement substitué,
   - lorsque G₂ représente un groupement G₂₁, X représentant un atome de soufre et B une chaîne éthylène, alors R₂₁ est différent d'un groupement aryle éventuellement substitué,
   le terme aryle désignant un groupement phényle ou naphtyle,
   le terme hétéroaryle désignant un groupement mono ou bicyclique de 4 à 11 chaînons saturé ou insaturé contenant de 1 à 4 hétéroatomes choisis parmi azote, oxygène et soufre, par exemple un groupement furyle, pyridyle, thiényle, ...
   étant entendu que :
   - le terme éventuellement substitué affectant les expressions alkyle, alkényle, alkynyle, cycloalkyle, signifie que ces groupements peuvent être substitués par un ou plusieurs atomes d'halogène, ou groupements cycloalkyle (C₃-C₇), hydroxy, alkoxy (C₁-C₆) linéaire ou ramifié, aryle éventuellement substitué, hétéroaryle éventuellement substitué,
   - le terme éventuellement substitué affectant les expressions aryle, biphényle, et hétéroaryle signifie que ces groupements peuvent être substitués par un ou plusieurs atomes d'halogène, ou groupements alkyle (C₁-C₆) linéaire ou ramifié, trihalogénoalkyle (C₁-C₆) linéaire ou ramifiés, hydroxy, alkoxy (C₁-C₆) linéaire ou ramifié, nitro, amino (éventuellement substitué par un ou deux groupements alkyle (C₁-C₆) linéaire ou ramifié identiques ou différents), alkylcarbonyle (C₁-C₆) linéaire ou ramifié, cyano, carboxy, ou aminocarbonyle (éventuellement substitué par un ou deux groupements alkyle (C₁-C₆) linéaires ou ramifié, identiques ou différents),
leurs énantiomères, diastéréoisomères, ainsi que leurs sels d'addition à un acide ou à une base phamaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Préférentiellement, l'invention concerne des composés benzo[*b*]furaniques ou benzo[*b*] thiophéniques.

De façon préférentielle dans les composés de formule (I), B représente une chaîne alkylène (C₁-C₆) linéaire ou ramifié, plus particulièrement une chaîne éthylène.

Les composés préférés de l'invention sont ceux pour lesquels G₁₁ représente un atome d'hydrogène.

D'autres composés préférés de l'invention sont ceux pour lesquels G₁ et R représentent chacun un atome d'hydrogène.

Le groupement R présent dans les composés de formule (I) est préférentiellement rattaché à la position 5 du noyau hétérocyclique.

Le groupement G₁₁ présent dans les composés de formule (I) est préférentiellement rattaché en position 5 du noyau hétérocyclique.

Les groupements R préférés de l'invention sont choisis parmi un atome d'hydrogène, un radical R' ou OR' dans lesquels R' représente un groupement alkyle (C₁-C₆) linéaire ou ramifié, alkényle (C₂-C₆) linéaire ou ramifié, ou alkynyle (C₂-C₆) linéaire ou ramifié. Plus particulièrement R' représente un groupement alkyle (C₁-C₆) linéaire ou ramifié, par exemple un groupement méthyle ou éthyle.

Un aspect avantageux de l'invention concerne les composés de formule (I) pour lesquels G₁ représente un groupement aryle éventuellement substitué, hétéroaryle éventuellement substitué, ou un groupement alkyle (C₁-C₆) linéaire ou ramifié substitué par un groupement choisi parmi cycloalkyle (C₃-C₇), aryle éventuellement substitué, et hétéroaryle éventuellement substitué. Plus particulièrement, G₁ représente un groupement méthylène substitué par un groupement phényle éventuellement substitué.

Un autre aspect avantageux de l'invention concerne les composés de formule (I) pour lesquels G₁₁ représente un groupement aryle éventuellement substitué, hétéroaryle éventuellement substitué, ou un groupement alkyle (C₁-C₆) linéaire ou ramifié substitué par un groupement choisi parmi cycloalkyle (C₃-C₇), aryle éventuellement substitué, et hétéroaryle éventuellement substitué, tandis que G₁ représente un atome d'hydrogène.

Dans les groupements G₂ préférés de l'invention, X représente un atome d'oxygène, R₂ représente un atome d'hydrogène et R₂₀, R₂₁, R₂₂ sont choisis parmi les groupements alkényle (C₂-C₆) linéaire ou ramifié, et alkyle (C₁-C₆) linéaire ou ramifié éventuellement substitué par un ou plusieurs atomes d'halogène.
Préférentiellement, G₂ représente un groupement G₂₀.

Un aspect très avantageux de l'invention concerne les composés de formule (I) pour lesquels la liaison 2-3 est insaturée, B représente une chaîne éthylène, R rattaché en position 5 du noyau hétérocyclique représente un atome d'hydrogène ou un groupement R' ou OR' dans lesquels R' est un groupement alkyle (C₁-C₆) linéaire ou ramifié, G₁₁ représente un atome d'hydrogène, G₁ représente un groupement aryle éventuellement substitué, hétéroaryle éventuellement substitué, ou un groupement alkyle (C₁-C₆) linéaire ou ramifié substitué par un groupement cycloalkyle (C₃-C₇), aryle éventuellement substitué, ou par a groupement hétéroaryle éventuellement substitué, et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par a ou plusieurs atomes d'halogène.

Un autre aspect très avantageux de l'invention concerne les composés de formule (I) pour lesquels la liaison 2-3 est insaturée, B représente une chaîne éthylène, R et G₁ représentent chacun un atome d'hydrogène, G₁₁ rattaché à la position 5 du noyau hétérocyclique représente un groupement aryle éventuellement substitué, hétéroaryle éventuellement substitué, ou un groupement alkyle (C₁-C₆) linéaire ou ramifié substitué par un groupement aryle éventuellement substitué ou hétéroaryle éventuellement substitué, et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par un ou plusieurs atomes d'halogène.

Parmi les composés préférés de l'invention, on peut citer plus particulièrement les :
- N-{2-[5-méthoxy-2-(3-méthoxybenzyl)-3-benzo[*b*]furyl]éthyl}acétamide
- N-{2-[5-méthoxy-2-(3-trifluorométhylbenzyl)-3-benzo[*b*]furyl]éthyl}acétamide
- N-[2-(2-benzyl-5-méthoxy-3-benzo[*b*]furyl]éthyl}acrylamide
- N-{2-[5-(2-méthoxyphényl)-3-benzo[*b*]furyl]éthyl}acétamide

L'invention concerne également le procédé de préparation des composés de formule (I) caractérisé en ce que l'on utilise comme matière première un dérivé de formule (II) : 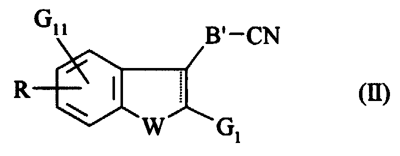 dans laquelle R, W, G₁ et G₁₁ sont tels que définis dans la formule (I), et B' représente une chaîne alkylène (C₀-C₅) linéaire ou ramifié éventuellement substitué par un ou plusieurs groupements alkyle(C₁-C₆) linéaire ou ramifié, hydroxy, alkoxy (C₁-C₆) linéaire ou ramifié, carboxy, ou alkoxycarbonyle (C₁-C₆) linéaire ou ramifié,
que l'on soumet :
↪ soit à une réaction de réduction qui peut être une hydrogénation catalytique ou une réduction chimique (par exemple en utilisant le complexe borane-tétrahydrofurane), pour conduire au composé de formule (III) : 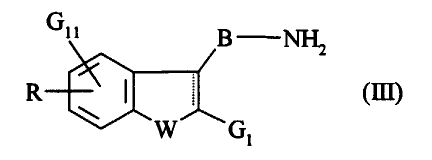 dans laquelle R, W, G₁ et G₁₁ sont tels que définis précédemment, et B est tel que défini dans la formule (I),
   a) qui peut être soumis à l'action d'un chlorure d'acyle de formule (IV) 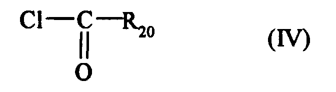 dans laquelle R₂₀ est tel que défini dans la formule (I),
      pour conduire à un composé de formule (I/a) : 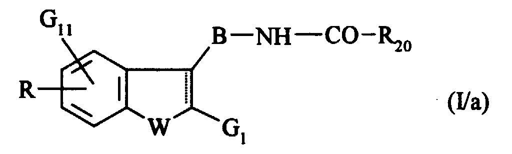 cas particulier des composés de formule (I) pour lequel R, W, B, G₁, G₁₁ et R₂₀ sont tels que définis précédemment,
      composé I/a qui peut être, si on le désire, soumis à une réaction d'alkylation classique en utilisant comme réactif un composé de formule P-R'₂, dans laquelle P représente un groupe partant (tel qu'un halogène ou un groupement tosyle) et R'₂, différent d'un atome d'hydrogène, a la même signification que R₂ dans la formule (I), ou bien en utilisant un dialkylsulfate, pour conduire à un composé de formule (I/b) : 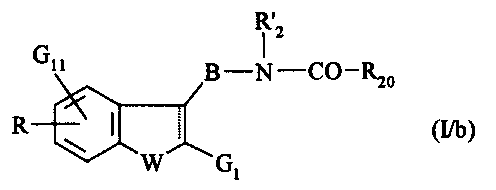 cas particulier des composés de formule (I) pour lequel R, W, B, G₁, G₁₁, R'₂ et R₂₀ sont tels que définis précédemment,
      composés (I/a) et (I/b) qui peuvent être soumis à un agent de thionation, par exemple le réactif de Lawesson, pour obtenir un composé de formule (I/c) : 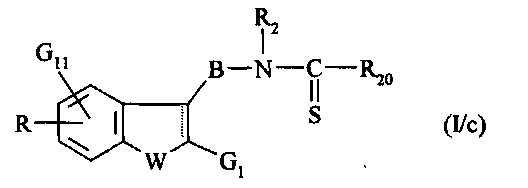 cas particulier des composés de formule (I) pour lequel R, W, B, G₁, G₁₁, R₂ et R₂₀ sont tels que définis précédemment,
   b) ou bien qui peut être soumis à l'action d'un iso(thio)cyanate de formule (V) :

      X=C=N-R₂₁ (V)

      dans laquelle X et R₂₁ sont tels que définis dans la formule (I),
      pour conduire à un composé de formule (I/d) : 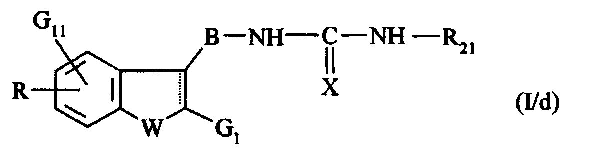 cas particulier des composés de formule (I) pour lequel R, W, B, G₁, G₁₁, X et R₂₁ sont tels que définis précédemment,
      composé (I/d) qui peut être soumis à une réaction d'alkylation en utilisant un agent classique d'alkylation de formule P-R'₂, dans laquelle P est un groupe partant (par exemple un atome d'halogène ou un groupement tosyle) et R'₂, différent d'un atome d'hydrogène, a la même signification que R₂ dans la formule (I), ou bien en utilisant un dialkylsulfate, pour conduire à un composé de formule (I/e) : 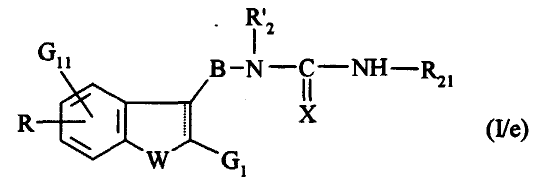 cas particulier des composés de formule (I) pour lequel R, W, B, G₁, G₁₁, R'₂, X et R₂₁ sont tels que définis précédemment,
↪ soit à une réaction d'hydrolyse en milieu acide ou basique pour conduire à l'acide carboxylique correspondant, de formule (VI) : 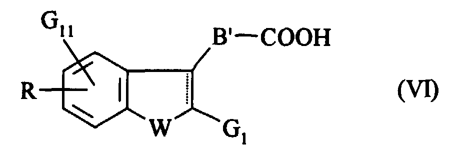 dans laquelle R, W, G₁ et G₁₁ ont la même signification que dans la formule (I), et B' est tel que défini précédemment,
   que l'on fait réagir, après formation éventuellement du chlorure d'acide correspondant, avec un composé de formule (VII): 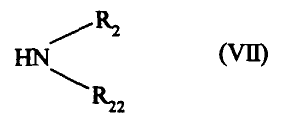 dans laquelle R₂ et R₂₂ sont tels que définis dans la formule (I),
   pour conduire à un composé de formule (I/f) : 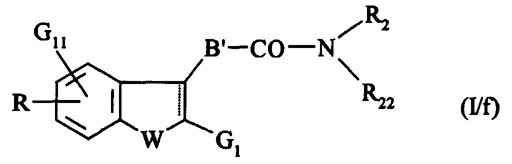 cas particulier des composés de formule (I) pour lequel R, W, B^{'}, G₁, G₁₁, R₂ et R₂₂ sont tels que définis précédemment,
   composé de formule (I/f) qui peut, si on le désire, être soumis à un agent de thionation, par exemple le réactif de Lawesson, pour conduire à un composé de formule (I/g) : 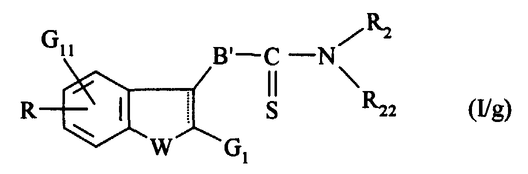 cas particulier des composés de formule (I) pour lequel R W, B', G₁, G₁₁, R₂ et R₂₂ sont tels que définis précédemment,
   composés de formule (I/a), (I/b), (I/c), (I/d), (I/e), (I/f), (I/g) qui lorsque R représente un groupement O-Alk (-Alk représentant un groupement alkyle (C₁-C₆) linéaire ou ramifié) et G₁₁ un groupement G'₁₁ ne pouvant former avec R un cycle, peuvent être, lorsque cela est compatible avec les substituants présents sur la molécule, traités par le tribromure de bore pour conduire au composé hydroxylé correspondant, de formule (VIII) : 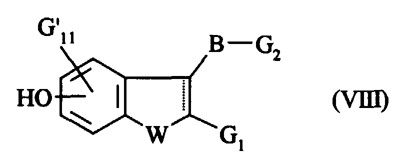 dans laquelle W, G₁, B et G₂ sont tels que définis dans la formule (I), et G'₁₁ a la même définition que G₁₁ dans la formule (I),
   composé de formule (VIII), dont la fonction hydroxyle peut être :
   a) soit transformée en trifluorométhanesulfonate, en utilisant par exemple le phényl bis(trifluorométhanesulfonimide) en milieu basique, pour conduire au composé de formule (IX): 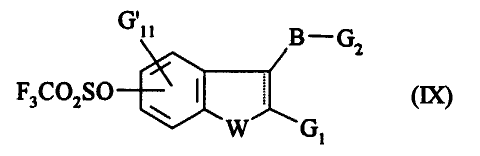 dans laquelle W, G₁, G'₁₁, B et G₂ sont tels que définis précédemment, que l'on peut transformer, par l'intermédiaire d'une réaction catalysée par un dérivé du palladium (O) en utilisant comme réactif un dérivé de l'acide borique (R'B(OH)₂) ou un dérivé de l'étain (R'SnBu₃), pour lesquels R' est tel que défini dans la formule (I), pour obtenir un composé de formule (I/h) : 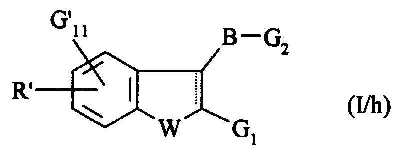 cas particulier des composés de formule (I) pour lequel R', W, G₁, G'₁₁, B et G₂ sont tels que définis précédemment,
   b) soit soumise à une réaction de O-substitution, en milieu basique, en utilisant comme réactif le dérivé halogéné approprié, pour conduire à un composé de formule (I/i) : 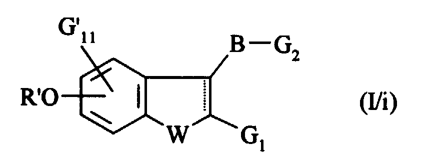 cas particulier des composés de formule (I) pour lequel R', W, G₁, G'₁₁, B et G₂ sont tels que définis précédemment,
   c) soit, lorsque G'₁₁ représente un atome d'hydrogène, soumise à une réaction de O-substitution pour conduire à un composé de formule (X) : 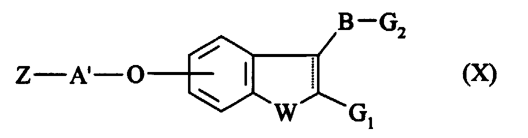 dans laquelle W, G₁, G₂ et B sont tels que définis précédemment, A' représente une chaîne alkylène (C₁-C₃), alkénylène (C₂-C₃) ou alkylène (C₂-C₃), et Z représente une fonction réactive (par exemple un acide carboxylique ou un ester) ou bien forme avec A' une liaison double ou triple,
      qui peut être cyclisé, par l'intermédiaire d'une réaction de substitution électrophile intra-moléculaire lorsque Z représente une fonction réactive ou par l'intermédiaire d'un réarrangement de claisen lorsque Z forme avec A' une liaison double ou triple, pour conduire à un composé de formule (I/k) : 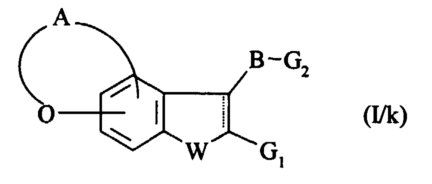 cas particulier des composés de formule (I) pour lequel W, G₁, G₂, et B sont tels que définis précédemment, et A représente une chaîne alkylène (C₂-C₄), alkénylène (C₃-C₄), alkynylène (C₃-C₄), ou une chaîne alkylène (C₂-C₄) substituée par un groupement oxo, ce dernier pouvant être, si on le désire, transformé en un groupement hydroxy, alkoxy (C₁-C₆) linéaire ou ramifié, alkyle (C₁-C₆) linéaire ou ramifié,
      composés (I/a) à (I/k) et (IX) formant la totalité des composés de formule (I),
      - que l'on purifie éventuellement selon une technique classique de purification,
      - dont on sépare éventuellement les énantiomères selon une technique classique de séparation,
      - et que l'on transforme, le cas échéant, en leurs sels d'addition à un acide ou à une base pharmaceutiquement acceptable.

Un des groupements G₁ ou G₁₁ tels que définis dans la formule (I) peut être transformé, lorsque cela est utile dans le but de simplifier le procédé ci-dessus, en un autre groupement représenté dans la description de G₁ ou de G₁₁ dans la formule (I), en utilisant les réactions classiques de la chimie organique.

Les matières premières utilisées dans le procédé précédemment décrit sont soit commerciales, soit aisément accessibles à l'homme de métier selon des procédés bien connus dans la littérature.

Par exemple, les composés de formule (II) telle que définie précédemment peuvent être obtenus à partir du composé carbonylé correspondant de formule (XI) : 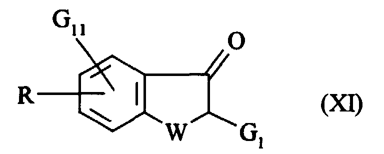 dans laquelle R, W, G₁ et G₁₁ sont tels que définis dans la formule (I), par une réaction de Wittig, d'Horner Emmons ou bien de Knoevenagel, en fonction des réactifs disponibles.

Lorsque cela peut se révéler avantageux, un composé de formule (VI/a) : 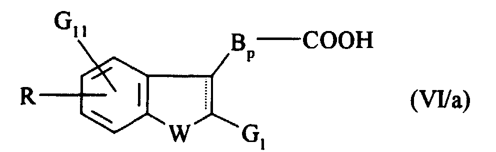 cas particulier des composés de formule (VI), dans lequel R, W, G₁ et G₁₁ sont tels que définis dans la formule (I) et Bₚ représente une chaîne comportant p atome de carbone telle que définie pour B dans la formule (I), p étant un entier compris entre 0 et 5,
peut subir une suite de réactions dans le but d'homologuer la chaîne carbonée portant la fonction acide, cette suite de réactions se décomposant en une réduction de l'acide en alcool, transformation de l,hydroxyle en groupe partant, et substitution de ce groupe par un dérivé cyané, pour conduire à un composé de formule (II/a) : 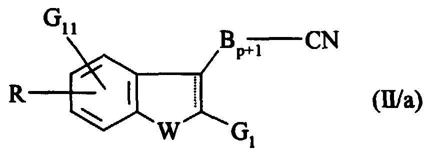 cas particulier des composés de formule (II) pour lequel R, W, G₁, G₁₁ et Bₚ sont tels que définis précédemment.

Les composés de formule (XI) telle que définie précédemment sont soit connus de l'homme de métier, ou peuvent être obtenus à partir de dérivés commerciaux ou décrits dans la littérature, de formule (XII) : 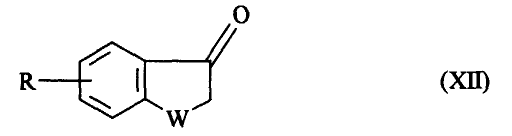 dans laquelle R et W sont tels que définis dans la formule (I),
- soit, lorsque G₁₁ représente un atome d'hydrogène par une réaction de condensation d'un aldéhyde approprié en milieu basique, (Tetrahedron lett., 1992, 32, 5937) ou bien par l'intermédiaire d'un organolithien condensé sur un électrophile approprié,
- soit, lorsque G₁ représente un atome d'hydrogène par des réactions de substitutions électrophiles ou nucléophiles en diverses positions du noyau aromatique.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardio-vasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l,invention, mais ne la limitent en aucune façon. Les structures des composés décrits ont été confirmées par des techniques spectroscopiques usuelles.

### Préparation A : 2-(5-Méthoxy-2-phényl-3-benzo[b]furyl)acétonitrile

### Stade a : 5-Méthoxy-3-méthyl-2-phényl-benzo[b]furane

Un mélange de 4,5 mmol (1,15 g) de 1-(4-méthoxyphénoxy)-1-phénylacétone et de 11,5 g d'acide polyphosphorique est chauffé à 50°C, sous agitation vigoureuse pendant 30 minutes. Après refroidissement, le mélange réactionnel est versé lentement sur de la glace, puis extrait à l'acétate d'éthyle. La phase organique est ensuite lavée à l'eau, séchée, concentrée et purifiée par chromatographie sur gel de silice en utilisant comme éluant un mélange éther de pétrole/toluène, 8/2, pour conduire au composé attendu.
Point de fusion : 106 - 107°C
   (réf : J-Chem. Soc. (C), 1970, p.155)

### Stade b : 2-(5-Méthoxy-2-phényl-3-benzo[b]furyl)acétonitrile

A une solution de 1,46 mmol (0,35 g) de composé décrit au stade précédent dans 10 ml de tétrachlorure de carbone, sont ajoutées 0,07 mmol (12 mg) d'AIBN et 1,46 mmol (0,26 g) de N-bromosuccinimide. La réaction est chauffée à reflux pendant 15 minutes à l'abri de la lumière. Après refroidissement le succinimide formé est filtré, et le filtrat concentré. Le résidu obtenu est dissous dans 2 ml de diméthylsulfoxyde et ajouté à une solution de 1.8 mmol (0.08 g) de cyanure de sodium dans 2 ml de diméthylsulfoxyde. Le milieu réactionnel est chauffé à 60°C pendant 1 heure, puis hydrolysé après refroidissement. Après extraction à l'acétate d'éthyle, la phase organique est lavée à l'eau, séchée, concentrée et purifiée par chromatographie sur gel de silice a utilisant comme éluant un mélange cyclohexane / acétate d'éthyle, 8/2, pour conduire au composé attendu.
Point de fusion : 106 - 107°C

### Préparation B : 2-(2-Benzyl-5-méthoxy-3-benzo[b]furyl)acétonitrile

### Stade a : 5-Méthoxy-2-(1-phénylméthylidène)-2,3-dihydro-3-benzo[b]furanone

A une solution de 30,5 mmol (5 g) de 5-méthoxy-3-benzo[*b*]furanone dans 150 ml de dichlorométhane sont ajoutées 36,5 mmol (3,9 g) de benzaldéhyde. Sous agitation vigoureuse et à température ambiante, sont alors ajoutés 25 g d'alumine basique. L'agitation est maintenue à température ambiante pendant 30 minutes. Le milieu est ensuite filtré, le filtrat concentré, et le résidu obtenu lavé par un minimum d'éthanol pour conduire au composé attendu.
Point de fusion : 134°C

### Stade b : 2-Benzyl-5-méthoxy-2,3-dihydro-3-benzo[b]furanone

Une solution de 25,8 mmol (6,5 g) du composé décrit au stade précédent dans un mélange de 400 ml de méthanol et 100 ml de dioxane est agitée 1h30 à température ambiante, sous pression atmosphérique d'hydrogène, en présence de 0,65 g de Palladium sur charbon. Après filtration du catalyseur, le filtrat est concentré pour conduire au composé attendu.

### Stade c : 2-(2-Benzyl-5-méthoxy-3-benzo[b]furyl)acétonitrile

Sous atmosphère inerte 38,4 mmol (1,3 g) d'hydrure de sodium à 60 % dans l'huile, sont mises en suspension dans 80 ml de tétrahydrofurane anhydre. A une température de -10°C, une solution de 38,4 mmol (6,8 g) de cyanométhylphosphonate de diéthyle dans 40 ml de tétrahydrofurane est ajoutée goutte à goutte. Après 15 minutes d'agitation à -10°C, 30 ml d'hexaméthylphosphoramide, et une solution de 25,6 mmol (6,5 g) du composé décrit au stade précédent dans 130 ml de tétrahydrofurane sont ajoutés successivement. Le milieu réactionnel a agité 2h30 à -10°C, puis hydrolysé. Après extraction à l'acétate d'éthyle, les phases organiques regroupées sont lavées par une solution d'acide chlorhydrique 1M, puis par une solution saturée de chlorure de sodium. Après séchage et évaporation des solvants, le résidu obtenu est purifié par chromatographie sur gel de silice en utilisant comme éluant un mélange acétate d'éthyle / éther de pétrole, 2/8, pour conduire au composé attendu.
Point de fusion : 69 - 70°C

### Préparation C : 2-[5-Méthoxy-2-(2-méthoxybenzyl)-3-benzo[b]furyl]acétonitrile

Le produit attendu est obtenu selon le procédé décrit dans la préparation B en remplaçant, au stade a, le benzaldéhyde par le 2-méthoxybenzaldéhyde.
Point de fusion : 122°C

### Préparation D : 2-[5-Méthoxy-2-(3-méthoxybenzyl)-3-benzo[b]furyl]acétonitrile

Le produit attendu est obtenu selon le procédé décrit dans la préparation B en remplaçant, au stade a, le benzaldéhyde par le 3-méthoxybenzaldéhyde.
Point de fusion : 81°C

### Préparation E : 2-[5-Méthoxy-2-(4-méthoxybenzyl))-3-benzo[b]furyl]acétonitrile

Le produit attendu est obtenu selon le procédé décrit dans la préparation B en remplaçant, au stade a, le benzaldéhyde par le 4-méthoxybenzaldéhyde.
Point de fusion : 83°C

### Préparation F : 2-[2-(3-Fluorobenzyl)-5-méthoxy-3-benzo[b]furyl]acétonitrile

Le produit attendu est obtenu selon le procédé décrit dans la préparation B en remplaçant, au stade a, le benzaldéhyde par le 3-fluorobenzaldéhyde.
Point de fusion : 67°C

### Préparation G : 2-[2-(3-Trifluorométhylbenzyl)-5-méthoxy-3-benzo[b]furyl] acétonitrile

Le produit attendu est obtenu selon le procédé décrit dans la préparation B en remplaçant, au stade a, le benzaldéhyde par le 3-(trifluorométhyl)benzaldéhyde.
Point de fusion : 116°C

### Préparation H : 2-[2-(2,6-Dichlorobenzyl)-5-méthoxy-3-benzo[b]furyl]acétonitrile

Le produit attendu est obtenu selon le procédé décrit dans la préparation B en remplaçant le benzaldéhyde par le 2,6-dichlorobenzaldéhyde.
Point de fusion: 184°C

### Préparation I : 2-(2-Furfuryl-5-méthoxy-3-benzo[b]furyl)acétonitrile

Le produit attendu est obtenu selon le procédé décrit dans la préparation B en remplaçant le benzaldéhyde par le 2-furaldéhyde.

### Préparation J : 2-[5-Méthoxy-2-(2-thiénylméthyl)-3-benzo[b]furyl]acétonitrile

Le produit attendu est obtenu selon le procédé décrit dans la préparation B en remplaçant le benzaldéhyde par le 2-thiophènecarboxaldéhyde.

### Préparation K : 2-[5-Méthoxy-2-(3-pyridylméthyl)-3-benzo[b]furyl]acétonitrile

Le produit attendu est obtenu selon le procédé décrit dans la préparation B en remplaçant, au stade a, le benzaldéhyde par le 3-pyridinecarboxaldéhyde.
Point de fusion : 123°C

### Préparation L : 2-[5-Méthoxy-2-(3-phénylpropyl)-3-benzo[b]furyl]acétonitrile

Le produit attendu est obtenu selon le procédé décrit dans la préparation B en remplaçant, au stade a, le benzaldéhyde par l'aldéhyde cinamique.

### Préparation M : 2-(5-Méthoxy-2-propyl-3-benzo[b]furyl)acétonitrile

Le produit attendu est obtenu selon le procédé décrit dans la préparation B en remplaçant, au stade a, le benzaldéhyde par le l'acroléïne.

### Préparation N : 2-(2-Benzyl-5-éthyl-3-benzo[b]furyl)acétonitrile

Le produit attendu est obtenu selon le procédé décrit dans la préparation B en remplaçant, au stade a, la 5-méthoxy-2,3-dihydro-3-benzo[*b*]furanone par la 5-éthyl-2,3-dihydro-3-benzo[*b*]furanone.

### Préparation O : 2-[5-Ethyl-2-(3-méthoxybenzyl))-3-benzo[b]furyl]acétonitrile

Le produit attendu est obtenu selon le procédé décrit dans la préparation D en utilisant comme produit de départ la 5-éthyl-2,3-dihydro-3-benzo[*b*]furanone.

### Préparation P : 2-[5-Ethyl-2-(3-fluorobenzyl)-3-benzo[b]furyl]acétonitrile

Le produit attendu est obtenu selon le procédé décrit dans la préparation F en utilisant comme produit de départ la 5-éthyl-2,3-dihydro-3-benzo[*b*]furanone.

### Préparation Q : 2-(5-Ethyl-2-furfuryl-3-benzo[b]furyl)acétonitrile

Le produit attendu est obtenu selon le procédé décrit dans la préparation I en utilisant comme produit de départ la 5-éthyl-2,3-dihydro-3-benzo[*b*]furanone.

### Préparation R : 2-(2-Cyclohexylméthyl-5-méthoxy-3-benzo[b]furyl)acétonitrile

Le produit attendu est obtenu selon le procédé décrit dans la préparation B en remplaçant, au stade a, le benzaldéhyde par le cyclohexanecarboxaldéhyde.
Point de fusion : 73°C

### Préparation S : 3-(5-Méthoxy-2-furfuryl-3-benzo[b]furyl)butanenitrile

Le produit attendu est obtenu par homologation de deux carbones de la chaîne portant la fonction nitrile, en utilisant comme produit de départ le composé décrit dans la préparation I.

### Préparation T : Acide 4-(5-méthoxy-2-furfuryl-3-benzo[b]furyl)butanoïque

Une solution de 3,5 mmol (1 g) du composé décrit dans la préparation S, dans 10 ml de méthanol est chauffée à reflux en présence de 10 ml d'une solution de soude 6M. Après refroidissement, le milieu réactionnel est acidifié par une solution d'acide chlorhydrique 6M et le précipité formé est filtré. Après recristallisation dans le toluène, le composé du titre est obtenu.

### Préparation U : 2-(5-Méthoxy-2-phényl-3-benzo[b]thiényl)acétonitrile

Le produit attendu est obtenu selon le procédé décrit dans la préparation A en remplaçant la 1-(4-méthoxyphénoxy)-1-phénylacétone par la 1-[(4-méthoxyphényl)sulfanyl]-1-phénylacétone.

### Préparation V : 2-(2-Benzyl-5-méthoxy-3-benzo[b]thiényl)acétonitrile

Le produit attendu est obtenu selon le procédé décrit dans la préparation B en remplaçant la 5-méthoxy-2,3-dihydro-3-benzo[*b*]furanone par la 5-méthoxy-2,3-dihydro-3-benzo[*b*] thiophénone.

### Préparation W : 2-[5-Méthoxy-2-(3-méthoxybenzyl)-3-benzo[b]thiényl]acétonitrile

Le produit attendu est obtenu selon le procédé décrit dans la préparation D en utilisant comme produit de départ la 5-méthoxy-2,3-dihydro-3-benzo[*b*]thiophénone.

### Préparation X : 2-[2-(3-Fluorobenzyl)-5-méthoxy-3-benzo[b]thiényl]acétonitrile

Le produit attendu est obtenu selon le procédé décrit dans la préparation F en utilisant comme produit de départ la 5-méthoxy-2,3-dihydro-3-benzo[*b*]thiophénone.

### Préparation Y : 2-[5-Méthoxy-2-(2-thiénylméthyl)-3-benzo[b]thiényl]acétonitrile

Le produit attendu est obtenu selon le procédé décrit dans la préparation J en utilisant comme produit de départ la 5-méthoxy-2,3-dihydro-3-benzo[*b*]thiophénone.

### Préparation Z : 2-[5-Méthoxy-2-(3-pyridylméthyl)-3-benzo[b]thiényl]acétonitrile

Le produit attendu est obtenu selon le procédé décrit dans la préparation K en utilisant comme produit de départ la 5-méthoxy-2,3-dihydro-3-benzo[*b*]thiophénone.

### Préparation AA : 2-(5-Méthoxy-2-propyl-3-benzo[b]thiényl)acétonitrile

Le produit attendu est obtenu selon le procédé décrit dans la préparation M en utilisant comme produit de départ la 5-méthoxy-2,3-dihydro-3-benzo[*b*]thiophénone.

### Préparation AB : 2-(2-Cyclohexylméthyl-5-méthoxy-3-benzo[b]thiényl)acétonitrile

Le produit attendu est obtenu selon le procédé décrit dans la préparation R en utilisant comme produit de départ la 5-méthoxy-2,3-dihydro-3-benzo[*b*]thiophénone.

### Préparation AC : 2-(2-Benzyl-5-éthyl-3-benzo[b]thiényl)acétonitrile

Le produit attendu est obtenu selon le procédé décrit dans la préparation N en utilisant comme produit de départ la 5-éthyl-2,3-dihydro-3-benzo[*b*]thiophénone.

### Préparation AD : 2-[2-(3-Chlorobenzyl)-5-méthoxy-3-benzo[b]furyl]acétonitrile

Le produit attendu est obtenu selon le procédé décrit dans la préparation B en remplaçant, au stade a, le benzaldéhyde par le 3-chlorobenzaldéhyde.
Point de fusion : 82°C

### Préparation AE : 2-[2-(3,5-Bistrifluorométhylbenzyl)-5-méthoxy-3-benzo[b]furyl] acétonitrile

Le produit attendu est obtenu selon le procédé décrit dans la préparation B en remplaçant, au stade a, le benzaldéhyde par le 3,5-bistrifluorométhylbenzaldéhyde.
Point de fusion: 112°C

### Exemple 1 : N-[2-(5-Méthoxy-2-phényl-3-benzo[b]furyl)éthyl]acétamide

### Stade a : 3-(5-Méthoxy-2-phényl-3-benzo[b]furyl)éthylamine, chlorhydrate

Sous atmosphère inerte, une solution de 6,84 mmol (1,8 g) du composé décrit dans la préparation A, dans 15 ml de tétrahydrofurane est ajoutée à 20,5 mmol (20 ml) de complexe borane-tétrahydrofurane 1M. Le milieu réactionnel est chauffé à reflux pendant 30 minutes, puis 13 ml d'acide chlorhydrique 6M sont ajoutés, et la réaction chauffée 30 minutes à reflux. Après refroidissement et évaporation des solvants, le résidu obtenu est lavé à l'éther. Le solide est filtré pour conduire au composé attendu.
Point de fusion : 255°C (décomposition)

### Stade b : N-[2-(5-Méthoxy-2-phényl-3-benzo[b]furyl)éthyl]acétamide

1,6 mmol (0,5 g) du composé décrit au stade précédent sont dissoutes dans un mélange de 8 ml de dichlorométhane et 4 ml d'eau. Sous agitation vigoureuse, 4,1 mmol (0,56 g) de carbonate de potassium et 1,81 mmol (0,13 ml) de chlorure d'acétyle sont ajoutées successivement à 0°C. La réaction est agitée à température ambiante pendant 2 heures. Le milieu réactionnel est extrait au dichlorométhane, les phases organiques, regroupées, concentrées et le résidu obtenu a recristallisé dans un mélange toluène/cyclohexane pour conduire au produit du titre.
Point de fusion: 113-115°C
Microanalyse élémentaire :

| | C | H | N |
|---|---|---|---|
| % Calc | 73,77 | 6,19 | 4,53 |
| %Trouvé | 73,87 | 6,15 | 4,50 |

### Exemple 2 : N-[2-(2-Benzyl-5-méthoxy-3-benzo[b]furyl)éthyl]acétamide

Le produit attendu est obtenu selon le procédé décrit dans l'exemple 1 en utilisant comme produit de départ le composé décrit dans la préparation B.
Point de fusion: 113-114°C
Microanalyse élémentaire :

| | C | H | N |
|---|---|---|---|
| % Calc | 74,45 | 6,54 | 4,33 |
| % Trouvé | 74,08 | 6,68 | 4,09 |

### Exemple 3 : N-{2-[5-Méthoxy-2-(2-méthoxybenzyl)-3-benzo[b]furyl]éthyl}acétamide

Le produit attendu est obtenu selon le procédé décrit dans l'exemple 1 en utilisant comme produit de départ le composé décrit dans la préparation C.
Point de fusion : 103°C
Microanalyse élémentaire :

| | C | H | N |
|---|---|---|---|
| % Calc | 71,37 | 6,56 | 3,96 |
| % Trouvé | 71,28 | 6,53 | 4,03 |

### Exemple 4 : N-{2-[5-Méthoxy-2-(3-méthoxybenzyl)-3-benzo[b]furyl]éthyl}acétamide

Le produit attendu est obtenu selon le procédé décrit dans l'exemple 1 en utilisant comme produit de départ le composé décrit dans la préparation D.
Point de fusion : 110°C
Microanalyse élémentaire :

| | C | H | N |
|---|---|---|---|
| % Calc | 71,37 | 6,56 | 3,96 |
| % Trouvé | 71,32 | 6,49 | 4,02 |

### Exemple 5 : N-{2-[5-Méthoxy-2-(4-méthoxybenzyl)-3-benzo[b]furyl]éthyl}acétamide

Le produit attendu est obtenu selon le procédé décrit dans l'exemple 1 en utilisant comme produit de départ le composé décrit dans la préparation E.
Point de fusion : 102°C
Microanalyse élémentaire :

| | C | H | N |
|---|---|---|---|
| % Calc | 71,37 | 6,56 | 3,96 |
| %Trouvé | 71,20 | 6,51 | 3,87 |

### Exemple 6 : N-{2-[(3-Fluorobenzyl)-5-méthoxy-3-benzo[b]furyl]éthyl}acétamide

Le produit attendu est obtenu selon le procédé décrit dans l'exemple 1 en utilisant comme produit de départ le composé décrit dans la préparation F.
Point de fusion : 137°C
Microanalyse élémentaire :

| | C | H | N | F |
|---|---|---|---|---|
| % Calc | 70,37 | 5,90 | 4,10 | 5,57 |
| % Trouvé | 70,33 | 5,92 | 3,97 | 5,30 |

### Exemple 7 : N-{2-[(3-Trifluorométhylbenzyl)-5-méthoxy-3-benzo[b]furyl]éthyl} acétamide

Le produit attendu est obtenu selon le procédé décrit dans l'exemple 1 en utilisant comme produit de départ le composé décrit dans la préparation G.
Point de fusion : 124°C
Microanalyse élémentaire :

| | C | H | N | F |
|---|---|---|---|---|
| % Calc | 64,45 | 5,15 | 3,58 | 14,56 |
| % Trouvé | 64,60 | 5,19 | 3,53 | 14,55 |

### Exemple 8 : N-{2-[(2,6-Dichlorobenzyl)-5-méthoxy-3-benzo[b]furyl]éthyl}acétamide

Le produit attendu est obtenu selon le procédé décrit dans l'exemple 1 en utilisant comme produit de départ le composé décrit dans la préparation H.
Point de fusion : 166°C
Microanalyse élémentaire :

| | C | H | N | Cl |
|---|---|---|---|---|
| % Calc | 61,24 | 4,88 | 3,57 | 18,07 |
| % Trouvé | 61,01 | 4,92 | 3,49 | 17,84 |

### Exemple 9 : N-[2-(Furfuryl-5-méthoxy-3-benzo[b]furyl)éthyl]acétamide

Le produit attendu est obtenu selon le procédé décrit dans l'exemple 1 en utilisant comme produit de départ le composé décrit dans la préparation I.

### Exemple 10 : N-{2-[5-Méthoxy-2-(2-thiénylméthyl)-3-benzo[b]furyl]éthyl}acétamide

Le produit attendu est obtenu selon le procédé décrit dans l'exemple 1 en utilisant comme produit de départ le composé décrit dans la préparation J.

### Exemple 11 : N-{2-[5-Méthoxy-2-(3-pyridylméthyl)-3-benzo[b]furyl]éthyl}acétamide

Le produit attendu est obtenu selon le procédé décrit dans l'exemple 1 en utilisant comme produit de départ le composé décrit dans la préparation K.
Point de fusion : 117°C

### Exemple 12 : N-{2-[5-Méthoxy-2-(3-phénylpropyl)-3-benzo[b]furyl]éthyl}acétamide

Le produit attendu est obtenu selon le procédé décrit dans l'exemple 1 en utilisant comme produit de départ le composé décrit dans la préparation L.
Point de fusion : 102°C
Microanalyse élémentaire :

| | C | H | N |
|---|---|---|---|
| % Calc | 75,19 | 7,17 | 3,99 |
| % Trouvé | 75,30 | 7,14 | 3,88 |

### Exemple 13 : N-[2-(5-Méthoxy-2-propyl-3-benzo[b]furyl)éthyl]acétamide

Le produit attendu est obtenu selon le procédé décrit dans l'exemple 1 en utilisant comme produit de départ le composé décrit dans la préparation M.

En procédant comme décrit dans l'Exemple 1, en utilisant au stade a, le composé décrit dans la Préparation B, et au stade b le chlorure d'acide approprié, on obtient les composés des exemples 14 à 19.

### Exemple 14 : N-[2-(2-Benzyl-5-méthoxy-3-benzo[b]furyl)éthyl]acrylamide

Point de fusion : 108-110°C
Microanalyse élémentaire :

| | C | H | N |
|---|---|---|---|
| % Calc | 75,20 | 6,31 | 4,18 |
| % Trouvé | 74,95 | 6,37 | 4,22 |

### Exemple 15 : N-[2-(2-Benzyl-5-méthoxy-3-benzo[b]furyl)éthyl]-3-buténamide

Point de fusion : 97°C
Microanalyse élémentaire :

| | C | H | N |
|---|---|---|---|
| % Calc | 75,62 | 6,63 | 4,01 |
| % Trouvé | 75,82 | 6,71 | 4,02 |

### Exemple 16 : N-[2-(2-Benzyl-5-méthoxy-3-benzo[b]furyl)éthyl]butanamide

Point de fusion : 86°C
Microanalyse élémentaire :

| | C | H | N |
|---|---|---|---|
| % Calc | 75,19 | 7,17 | 3,99 |
| % Trouvé | 74,82 | 7,17 | 3,94 |

### Exemple 17 : N-[2-(2-Benzyl-5-méthoxy-3-benzo[b]furyl)éthyl]-2-phénylacétamide

### Exemple 18 : N-[2-(2-Benzyl-5-méthoxy-3-benzo[b]furyl)éthyl]trifluoroacétamide

### Exemple 19 : N-[2-(2-Benzyl-5-méthoxy-3-benzo[b]furyl)éthyl]iodoacétamide

Point de fusion : 135-136°C
Microanalyse élémentaire :

| | C | H | N | I |
|---|---|---|---|---|
| % Calc | 53,46 | 4,48 | 3,11 | 21,26 |
| % Trouvé | 53,76 | 4,56 | 2,92 | 27,82 |

### Exemple 20 : N-[2-(2-Benzyl-5-éthyl-3-benzo[b]furyl)éthyl]acétamide

Le produit attendu est obtenu selon le procédé décrit dans l'exemple 1 en utilisant comme produit de départ le composé décrit dans la préparation N.
Point de fusion : 100-101°C
Microanalyse élémentaire :

| | C | H | N |
|---|---|---|---|
| % Calc | 78,47 | 7,21 | 4,36 |
| % Trouvé | 78,29 | 7,12 | 4,18 |

### Exemple 21 : N-{2-[5-Ethyl-2-(3-méthoxybenzyl-3-benzo[b]furyl]éthyl)acétamide

Le produit attendu est obtenu selon le procédé décrit dans l'exemple 1 en utilisant comme produit de départ le composé décrit dans la préparation O.
Point de fusion : 102°C
Microanalyse élémentaire :

| | C | H | N |
|---|---|---|---|
| % Calc | 75,19 | 7,17 | 3,99 |
| % Trouvé | 75,04 | 7,17 | 3,96 |

### Exemple 22 : N-{2-[5-Ethyl-2-(3-fluorobenzyl)-3-benzo[b]furyl]éthyl}acétamide

Le produit attendu est obtenu selon le procédé décrit dans l'exemple 1 en utilisant comme produit de départ le composé décrit dans la préparation P.

### Exemple 23 : N-[2-(5-Ethyl-2-furfuryl-3-benzo[b]furyl)éthyl]acétamide

Le produit attendu est obtenu selon le procédé décrit dans l'exemple 1 en utilisant comme produit de départ le composé décrit dans la préparation Q.

### Exemple 24 : N-[2-(2-Cyclohexylméthyl-5-méthoxy-3-benzo[b]furyl)éthyl]acétamide

Le produit attendu est obtenu selon le procédé décrit dans l'exemple 1 en utilisant comme produit de départ le composé décrit dans la préparation R.
Microanalyse élémentaire :

| | | | |
|---|---|---|---|
| | C | H | N |
| % Calc | 72,92 | 8,26 | 4,25 |
| % Trouvé | 72,85 | 8,12 | 4,08 |

### Exemple 25 : N-[2-(2-Benzyl-5-hydroxy-3-benzo[b]furyl)éthyl]acétamide

Une solution de 12,4 mmol (3,9 g) de complexe tribromure de bore-diméthylsulfure dans 45 ml de dichlorométhane est agitée 15 minutes à température ambiante. Une solution de 6,2 mmol (2 g) du composé décrit dans l'exemple 2 dans 30 ml de dichlorométhane est ajoutée au mélange précédent et la réaction est chauffée à reflux pendant 1 nuit. Après refroidissement et hydrolyse, le dichlorométhane est évaporé. Le milieu est extrait à l'acétate d'éthyle et la phase organique est lavée par une solution aqueuse de NaHCO₃ 1M puis par une solution aqueuse de soude 1M. La phase aqueuse ainsi obtenue est acidifiée puis extraite par l'acétate d'éthyle. La phase organique est séchée et concentrée pour conduire au composé du titre.

### Exemple 26 : N-[2-(2-Benzyl-5-pentyloxy-3-benzo[b]furyl)éthyl]acétamide

Une solution de 1,9 mmol (0,6 g) de composé décrit dans l'exemple 25 dans 25 ml d'acétone est chauffée à reflux pendant 15 minutes en présence de 3,8 mmol (0,5 g) de carbonate de potassium. Puis 3,8 mmol (0,5 ml) d'iodopentane sont ajoutées, et la réaction est chauffée à reflux pendant 1 nuit. Après refroidissement et filtration, le filtrat est concentré. Le résidu obtenu est repris dans un mélange acétate d'éthyle/eau et extrait. La phase organique est lavée par une solution de soude à 20 % puis par une solution aqueuse saturée de chlorure de sodium, séchée et concentrée pour conduire au composé attendu.
Point de fusion : 71°C
Microanalyse élémentaire :

| | C | H | N |
|---|---|---|---|
| % Calc | 75,96 | 7,70 | 3,69 |
| % Trouvé | 76,05 | 7,70 | 3,63 |

### Exemple 27 : N-[2-(2-Benzyl-5-hexyloxy-3-benzo[b]furyl)éthyl]acétamide

Le produit attendu est obtenu selon le procédé décrit dans l'exemple 26 en remplaçant l'iodopentane par le bromohexane.
Point de fusion : 64°C
Microanalyse élémentaire :

| | C | H | N |
|---|---|---|---|
| % Calc | 76,30 | 7,94 | 3,56 |
| %Trouvé | 76,15 | 7,93 | 3,58 |

### Exemple 28 : N-[2-(2-Benzyl-5-cyclopropylméthyloxy-3-benzo[b]furyl)éthyl] acétamide

Le produit attendu est obtenu selon le procédé décrit dans l'exemple 26 en remplaçant l'iodopentane par le chlorométhylclopropane.

### Exemple 29 : N-{2-[2-Benzyl-5-(2-propynyloxy)-3-benzo[b]furyl]éthyl}acétamide

Le produit attendu est obtenu selon le procédé décrit dans l'exemple 26 en remplaçant l'iodopentane par le bromure de propargyle.

### Exemple 30 : N-{2-[2-Benzyl-1-(7H-furo[3,2-f]chroményl)]éthyl}acétamide

Une solution de 8,6 mmol (3 g) de composé décrit dans l'exemple 29 dans 100 ml de triéthylèneglycol est chauffée à 280°C pendant 10 minutes. Après refroidissement, le milieu réactionnel est versé sur un mélange eau/glace. Après agitation, le précipité formé est filtré, et le solide obtenu lavé à l'eau, et recristallisé dans un mélange toluène/cyclohexane pour conduire au composé attendu.

### Exemple 31 : N-{2-[2-Benzyl-1-(8,9-dihydro-7H-furo[3,2-f]chroményl)]éthyl} acétamide

Une solution de 1,4 mmol (0,5 g) du composé décrit dans l'exemple 30 dans 25 ml de méthanol, en présence de 0,05 g de Nickel de Raney, est agitée pendant deux heures sous pression atmosphérique d'hydrogène. Le catalyseur est filtré et le solvant évaporé. Le résidu est recristallisé dans un mélange toluène/cyclohexane pour conduire au composé du titre.

### Exemple 32 : N-[2-(2-Benzyl-5-trifluorométhanesulfonyloxy-3-benzo[b]furyl)éthyl] acétamide

A une solution de 6,5 mmol (2 g) du composé décrit dans l'exemple 25 dans 100 ml de dichlorométhane sont ajoutés 6 ml de triéthylamine. Le milieu réactionnel est porté à reflux jusqu'à solubilisation, puis, 9,1 mmol (3,25 g) de phényl bis (trifluorométhanesulfonimide) et 7,2 mmol (1 g) de carbonate de potassium sont ajoutés. Après 4 heures à reflux, le milieu est lavé par 100 ml d'hydrogénocarbonate de sodium 1M puis par 100 ml d'acide chlorhydrique 1M. La phase organique est séchée, concentrée, et purifiée par chromatographie sur gel de silice en utilisant l'acétate d'éthyle comme éluant pour conduire au composé attendu.

### Exemple 33 : N-[2-(2-Benzyl-5-phényl-3-benzo[b]furyl)éthyl]acétamide

A une solution de 5,9 mmol (2,5 g) du composé décrit dans l'exemple 32 dans 25 ml de diméthoxyéthane, sous atmosphère inerte, sont ajoutées 8,9 mmol (1,2 g) d'acide phénylborique, 0,12 g de tétrakistriphénylphosphine palladium (0) et 0,6 g de chlorure de lithium. Le milieu réactionnel est agité 10 minutes puis 16 ml d'une solution molaire de carbonate de sodium et 12 ml d'éthanol absolu sont ajoutés. La réaction est chauffée à 90°C pendant 4 heures. Après refroidissement, 50 ml de carbonate de sodium 1M sont ajoutés, et le milieu est extrait deux fois par 50 ml de dichlorométhane. La phase organique est séchée, concentrée et purifiée par chromatographie sur gel de silice, en utilisant l'acétate d'éthyle comme éluant, pour conduire au composé attendu.

Les composés des exemples 34 à 35 sont obtenus en utilisant le procédé décrit dans l'exemple 33 et l'acide borique ou le dérivé de l'étain approprié.

### Exemple 34 : N-[2-(2-Benzyl-5-furyl-3-benzo[b]furyl)éthyl]acétamide

### Exemple 35 : N-[2-(2-Benzyl-5-vinyl-3-benzo[b]furyl)éthyl]acétamide

### Exemple 36 : N-[2-(2-Benzyl-3-benzo[b]furyl)éthyl]acétamide

Le produit attendu est obtenu selon le procédé décrit dans l'exemple 33 en remplaçant l'acide phénylborique par l'acide formique.

### Exemple 37 : N-[3-(2-Furfuryl-5-méthoxy-3-benzo[b]furyl)butyl]acétamide

Le produit attendu est obtenu selon le procédé décrit dans l'exemple 1 en utilisant comme produit de départ le composé décrit dans la préparation S.

### Exemple 38 : N-Méthyl-4-(2-furfuryl-5-méthoxy-3-benzo[b]furyl)butanamide

Une solution de 1,5 mmol (0,5 g) du composé décrit dans la préparation T, dans 10 M de dichlorométhane est agitée à -10°C pendant 20 mn. Sont ajoutées successivement, 2,31 mmol (0,23 g) de triéthylamine, 2,31 mmol (0,31 g) d'HOBT, 2,31 mmol (0,44 g) d'EDC. Le milieu réactionnel est agité 30 minutes à -10°C, et 3,1 mmol (0,20 g) de chlohydrate de méthylamine sont ajoutées. La réaction est agitée 2 heures à température ambiante. Le milieu est concentré, et le résidu est repris dans mélange acétate d'éthyle/eau. La phase organique est lavée par une solution d'acide chlorhydrique 0,1 M, rincée à l'eau, lavée par une solution de soude 1M puis rincée à l'eau. Après séchage et concentration, le résidu obtenu est recristallisé dans un mélange toluène/éther de pétrole pour conduire au produit du titre.

### Exemple 39 : N-[2-(4-Formyl-5-méthoxy-3-benzo[b]furyl)éthyl]acétamide

Le produit attendu est obtenu pu formylation du N-[2-(5-méthoxy-3-benzo[*b*]furyl)éthyl] acétamide, en utilisant le dichlorométhyl méthyl éther en présence de trichlorure d'aluminium.
*Remarque :* Au cours de ce procédé le composé formylé en position 6 est également isolé

### Exemple 40 : N-[2-(5-Méthoxy-4-trifluorométhanesulfonyloxy-3-benzo[b]furyl)éthyl] acétamide

### Stade a : N-[2-(4-Hydroxy-5-méthoxy-3-benzo[b]furyl)éthyl]acétamide

Le produit attendu est obtenu par réaction de Bayer Villiger à partir du composé décrit dans l'exemple 39.

### Stade b : N-[2-(5-Méthoxy-4-trifluorométhanesulfonyloxy-3-benzo[b]furyl)éthyl] acétamide

Le produit attendu est obtenu selon le procédé décrit dans l'exemple 32 à partir du composé décrit au stade précédent.

Les composés des exemples 41 à 44 sont obtenus selon le procédé décrit dans l'exemple 33, en utilisant comme produit de départ le composé décrit dans l'exemple 40 et comme réactif le dérivé de l'acide borique ou de l'étain approprié.

### Exemple 41 : N-[2-(5-Méthoxy-4-phényl-3-benzo[b]furyl)éthyl]acétamide

### Exemple 42 : N-{2-[5-Méthoxy-4-(3-trifluorométhylphényl)-3-benzo[b]furyl]éthyl} acétamide

### Exemple 43 : N-{2-[4-(2-Furyl)-5-méthoxy-3-benzo[b]furyl]éthyl}acétamide

### Exemple 44 : N-[2-(4-Benzyl-5-méthoxy-3-benzo[b]furyl)éthyl]acétamide

### Exemple 45 : N-[2-(6-Formyl-5-méthoxy-3-benzo[b]furyl)éthyl]acétamide

Le produit attendu est isolé au cours du procédé décrit dans l'exemple 39.

### Exemple 46 : N-[2-(5-Méthoxy-6-trifluorométhanesulfonyloxy-3-benzo[b]furyl)éthyl] acétamide

Le produit attendu est obtenu selon le procédé décrit dans l'exemple 40 en utilisant comme produit de départ le composé décrit dans l'exemple 45.

Les composés des exemples 47 à 50 sont obtenus selon le procédé décrit dans l'exemple 33, en utilisant comme produit de départ le composé décrit dans l'exemple 46 et comme réactif le dérivé de l'acide borique ou de l'étain approprié.

### Exemple 47 : N-[2-(5-Méthoxy-6-phényl-3-benzo[b]furyl)éthyl]acétamide

### Exemple 48 : N-{2-[5-Méthoxy-6-(3-trifluorométhylphényl)-3-benzo[b]furyl]ethyl} acétamide

### Exemple 49 : N-{2-[6-(2-Furyl)-5-méthoxy-3-benzo[b]furyl]éthyl}acétamide

### Exemple 50 : N-[2-(6-Benzyl-5-méthoxy-3-benzo[b]furyl)éthyl]acétamide

### Exemple 51 : N-[2-(5-Trifluorométhanesulfonyloxy-3-benzo[b]furyl)éthyl]acétamide

### Stade a : N-[2-(5-Hydroxy-3-benzo[b]furyl)éthyl]acétamide

Le produit attendu est obtenu selon le procédé décrit dans l'exemple 25 en utilisant comme produit de départ le N-[2-(5-méthoxy-3-benzo[*b*]furyl)éthyl]acétamide.

### Stade b : N-[2-(5-Trifluorométhanesulfonyloxy-3-benzo[b]furyl)éthyl]acétamide

Le produit attendu est obtenu selon le procédé décrit dans l'exemple 32 à partir du composé décrit au stade précédent.

Les composés des exemples 52 à 56 sont obtenus selon le procédé décrit dans l'exemple 33, en utilisant comme produit de départ le composé décrit dans l'exemple 51 et le dérivé de l'acide borique ou de l'étain approprié.

### Exemple 52 : N-[2-(5-Phényl-3-benzo[b]furyl)éthyl]acétamide

### Exemple 53 : N-[2-(5-Vinyl-3-benzo[b]furyl)éthyl]acétamide

### Exemple 54 : N-{2-[5-(2-Furyl)-3-benzo[b]furyl]éthyl}acétamide

### Exemple 55 : N-[2-(5-Benzyl-3-benzo[b]furyl)éthyl]acétamide

### Exemple 56 : N-{2-[5-(2-Méthoxyphényl)-3-benzo[b]furyl]éthyl}acétamide

### Exemple 57 : N-[2-(5-Méthoxy-2-furfuryl-3-benzo[b]furyl)éthyl]-N'-méthylurée

### Stade a : 2-(5-Méthoxy-2-furfuryl-3-benzo[b]furyl)éthylamine

Le produit attendu est obtenu selon le procédé décrit dans l'exemple 1, stade a, en utilisant comme produit de départ le composé décrit dans la préparation Q.

### Stade b : N-[2-(5-Méthoxy-2-furfuryl-3-benzo[b]furyl)éthyl]-N'-méthylurée

Le produit attendu est obtenu par action de l'isocyanate de méthyle sur le composé décrit au stade précédent.

### Exemple 58 : N-[2-(2-Benzyl)-5-méthoxy-3-benzo[b]furyl]éthyl}-N'-allylurée

Le produit attendu est obtenu selon le procédé décrit dans l'exemple 57, en utilisant comme produit de départ le composé décrit dans la préparation B, et en remplaçant l'isocyanate de méthyle par l'isocyanate d'allyle.

Les composés des exemples 59 à 63 sont obtenus en utilisant le réactif de Lawesson, à partir des produits décrits dans les exemples précédents.

### Exemple 59 : N-[2-(2-Benzyl-5-méthoxy-3-benzo[b]furyl)éthyl]thioacétamide

### Exemple 60 : N-[2-(5-méthoxy-2-phényl-3-benzo[b]furyl)éthyl]thioacétamide

### Exemple 61 : N-{2-[5-méthoxy-2-(3-méthoxybenzyl)-3-benzo[b]furyl]éthyl} thioacétamide

### Exemple 62 : N-{2-[5-Méthoxy-2-(3-pyridylméthyl)-3-benzo[b]furyl]éthyl} thioacétamide

### Exemple 63 : N-[2-(2-Benzyl-5-éthyl-3-benzo[b]furyl)éthyl]thioacétamide

### Exemple 64 : N-[2-(5-Méthoxy-2-phényl-3-benzo[b]thiényl)éthyl]acétamide

Le produit attendu est obtenu selon le procédé décrit dans l'exemple 1 en utilisant comme produit de départ le composé décrit dans la préparation U.

### Exemple 65 : N-[2-(2-Benzyl-5-méthoxy-3-benzo[b]thiényl)éthyl]acétamide

Le produit attendu est obtenu selon le procédé décrit dans l'exemple 1 en utilisant comme produit de départ le composé décrit dans la préparation V.

### Exemple 66 : N-{2-[5-Méthoxy-2-(3-méthoxybenzyl)-3-benzo[b]thiényl]éthyl} acétamide

Le produit attendu est obtenu selon le procédé décrit dans l'exemple 1 en utilisant comme produit de départ le composé décrit dans la préparation W.

### Exemple 67 : N-{2-[2-(3-Fluorobenzyl)-5-méthoxy-3-benzo[b]thiényl]éthyl}acétamide

Le produit attendu est obtenu selon le procédé décrit dans l'exemple 1 en utilisant comme produit de départ le composé décrit dans la préparation X.

### Exemple 68 : N-{2-[5-Méthoxy-2-(2-thiénylméthyl)-3-benzo[b]thiényl]éthyl} acétamide

Le produit attendu est obtenu selon le procédé décrit dans l'exemple 1 en utilisant comme produit de départ le composé décrit dans la préparation Y.

### Exemple 69 : N-{2-[5-Méthoxy-2-(3-pyridylméthyl)-3-benzo[b]thiényl]éthyl} acétamide

Le produit attendu est obtenu selon le procédé décrit dans l'exemple 1 en utilisant comme produit de départ le composé décrit dans la préparation Z.

### Exemple 70 : N-[2-(5-Méthoxy-2-propyl-3-benzo[b]thiényl)éthyl]acétamide

Le produit attendu est obtenu selon le procédé décrit dans l'exemple 1 en utilisant comme produit de départ le composé décrit dans la préparation AA.

### Exemple 71 : N-[2-(2-Cyclohexylméthyl-5-méthoxy-3-benzo[b]thiényl)éthyl]acétamide

Le produit attendu est obtenu selon le procédé décrit dans l'exemple 1 en utilisant comme produit de départ le composé décrit dans la préparation AB.

En utilisant le procédé décrit dans l'exemple 1, à partir du composé décrit dans la préparation V et en choisissant comme réactif le chlorure d'acide approprié, on obtient les composés des exemples 72 à 75.

### Exemple 72 : N-[2-(2-Benzyl-5-méthoxy-3-benzo[b]thiényl)éthyl]acrylamide

### Exemple 73 : N-[2-(2-Benzyl-5-méthoxy-3-benzo[b]thiényl)éthyl]butanamide

### Exemple 74 : N-[2-(2-Benzyl-5-méthoxy-3-benzo[b]thiényl)éthyl]-2-phénylacétamide

### Exemple 75 : N-[2-(2-Benzyl-5-méthoxy-3-benzo[b]thiényl)éthyl]iodoacétamide

### Exemple 76 : N-[2-(2-Benzyl-5-éthyl-3-benzo[b]thiényl)éthyl]acétamide

Le produit attendu est obtenu selon le procédé décrit dans l'exemple 1 en utilisant comme produit de départ le composé décrit dans la préparation AC.

### Exemple 77 : N-[2-(2-Benzyl-5-hydroxy-3-benzo[b]thiényl)éthyl]acétamide

Le produit attendu est obtenu selon le procédé décrit dans l'exemple 25 en utilisant comme produit de départ le composé décrit dans l'exemple 65.

### Exemple 78 : N-[2-(2-Benzyl-5-hexyloxy-3-benzo[b]thiényl)éthyl[acétamide

Le produit attendu est obtenu selon le procédé décrit dans l'exemple 27 en utilisant comme produit de départ le composé décrit dans l'exemple 77.

### Exemple 79 : N-{2-[2-Benzyl-5-(2-propynyloxy)-3-benzo[b]thiényl]éthyl}acétamide

Le produit attendu est obtenu selon le procédé décrit dans l'exemple 29 en utilisant comme produit de départ le composé décrit dans l'exemple 77.

### Exemple 80 : N-{2-[2-Benzyl-1-(7H-thiéno[3,2-f]chroményl)]éthyl}acétamide

Le produit attendu est obtenu selon le procédé décrit dans l'exemple 30 en utilisant comme produit de départ le composé décrit dans l'exemple 79.

### Exemple 81 : N-{2-[2-Benzyl-1-(8,9-dihydro-7H-thiéno[3,2-f]chroményl)]éthyl} acétamide

Le produit attendu est obtenu selon le procédé décrit dans l'exemple 31 en utilisant comme produit de départ le composé décrit dans l'exemple 80.

### Exemple 82 : N-[2-(2-Benzyl-5-trifluorométhanesulfonyloxy-3-benzo[b]thiényl)éthyl] acétamide

Le produit attendu est obtenu selon le procédé décrit dans l'exemple 32 en utilisant comme produit de départ le composé décrit dans l'exemple 77.

Les composés des exemples 83 à 85 sont obtenus selon le procédé décrit dans l'exemple 33, en utilisant comme produit de départ le composé décrit dans l'exemple 82 et comme réactif le dérivé de l'acide borique ou de l'étain approprié.

### Exemple 83 : N-[2-(2-Benzyl-5-phényl-3-benzo[b]thiényl)éthyl]acétamide

### Exemple 84 : N-{2-[2-Benzyl-5-(2-furyl)-3-benzo[b]thiényl]éthyl}acétamide

### Exemple 85 : N-[2-(2-Benzyl-5-vinyl-3-benzo[b]thiényl)éthyl]acétamide

### Exemple 86 : N-[2-(2-Benzyl-3-benzo[b]thiényl)éthyl]acétamide

Le produit attendu est obtenu selon le procédé décrit dans l'exemple 36 en utilisant comme produit de départ le composé décrit dans l'exemple 82.

### Exemple 87 : N-[2-(4-Formyl-5-méthoxy-3-benzo[b]thiényl)éthyl]acétamide

Le produit attendu est obtenu selon le procédé décrit dans l'exemple 39 en utilisant comme produit de départ le N-[2-(5-méthoxy-3-benzo[*b*]thiényl)éthyl]acétamide.
*Remarque :* Au cours de ce procédé le composé formylé en position 6 est également isolé

### Exemple 88 : N-[2-(5-Méthoxy-4-trifluorométhansulfonyloxy-3-benzo[b]thiényl) éthyl]acétamide

Le produit attendu est obtenu selon le procédé décrit dans l'exemple 40 en utilisant comme produit de départ le composé décrit dans l'exemple 87.

Les composés des exemples 89 à 91 sont obtenus selon le procédé décrit dans l'exemple 33, en utilisant comme produit de départ le composé décrit dans l'exemple 88, et comme réactif le dérivé de l'acide borique ou de l'étain approprié.

### Exemple 89 : N-[2-(5-Méthoxy-4-phényl-3-benzo[b]thiényl)éthyl]acétamide

### Exemple 90 : N-{2-[4-(2-Furyl)-5-méthoxy-3-benzo[b]thiényl]éthyl}acétamide

### Exemple 91 : N-[2-(4-Benzyl-5-méthoxy-3-benzo[b]thiényl)éthyl]acétamide

### Exemple 92 : N-[2-(6-Formyl-5-méthoxy-3-benzo[b]thiényl)éthyl]acétamide

Le produit attendu est isolé lors du procédé décrit dans l'exemple 87.

### Exemple 93 : N-[2-(5-Méthoxy-6-trifluorométhansulfonyloxy-3-benzo[b]thiényl) éthyl]acétamide

Le produit attendu est obtenu selon le procédé décrit dans l'exemple 40, en utilisant comme produit de départ le composé décrit dans l'exemple 92.

Les composés des exemples 94 à 96 sont obtenus selon le procédé décrit dans l'exemple 33, en utilisant comme produit de départ le composé décrit dans l'exemple 93, et comme réactif le dérivé de l'acide borique ou de l'étain approprié.

### Exemple 94 : N-[2-(5-Méthoxy-6-phényl-3-benzo[b]thiényl)éthyl]acétamide

### Exemple 95 : N-{2-[6-(2-Furyl)-5-méthoxy-3-benzo[b]thiényl]éthyl}acétamide

### Exemple 96 : N-[2-(6-Benzyl-5-méthoxy-3-benzo[b]thiényl)éthyl]acétamide

### Exemple 97 : N-[2-(5-Trifluorométhanesulfonyloxy-3-benzo[b]thiényl)éthyl]acétamide

Le produit attendu est obtenu selon le procédé décrit dans l'exemple 51, en utilisant comme produit de départ le N-[2-(5-méthoxy-3-benzo[*b*]thiényl)éthyl]acétamide.

Les composés des exemples 98 à 102 sont obtenus selon le procédé décrit dans l'exemple 33, en utilisant comme produit de départ le composé décrit dans l'exemple 97, et comme réactif le dérivé de l'acide borique ou de l'étain approprié.

### Exemple 98 : N-[2-(5-Phényl-3-benzo[b]thiényl)éthyl]acétamide

### Exemple 99 : N-[2-(5-Vinyl-3-benzo[b]thiényl)éthyl]acétamide

### Exemple 100 : N-[2-(5-Benzyl-3-benzo[b]thiényl)éthyl]acétamide

### Exemple 101 : N-{2-[5-(2-Furyl)-3-benzo[b]thiényl]éthyl}acétamide

### Exemple 102 : N-[2-(2-Benzyl-5-méthoxy-3-benzo[b]thiényl)éthyl]-N'-allylurèe

Le produit attendu est obtenu selon le procédé décrit dans l'exemple 57, en utilisant comme produit de départ le composé décrit dans la préparation V, en en remplaçant l'isocyanate de méthyle par l'isocyanate d'allyle.

Les composés des exemples 103 à 105 sont obtenus en utilisant le réactif de Lawesson, à partir des produits décrits dans les exemples précédents.

### Exemple 103 : N-[2-(5-Méthoxy-2-phényl-3-benzo[b]thiényl)éthyl]thioacétamide

### Exemple 104 : N-{2-[5-Méthoxy-2-(3-méthoxybenzyl)-3-benzo[b]thiényl]éthyl} thioacétamide

### Exemple 105 : N-[2-(2-Benzyl-5-éthyl-3-benzo[b]thiényl)éthyl]thioacétamide

En utilisant le procédé décrit dans l'exemple 1, et en remplaçant, au stade b, le chlorure d'acétyle par le chlorure de l'acide approprié, on obtient les composés des exemples 106 à 108.

### Exemple 106 : N-[2-(5-Méthoxy-2-phényl-3-benzo[b]furyl)éthyl]acrylamide

Point de fusion : 117°C
Microanalyse élémentaire :

| | C | H | N |
|---|---|---|---|
| % Calc | 74,75 | 5,96 | 4,36 |
| % Trouvé | 74,47 | 5,94 | 4,27 |

### Exemple 107 : N-[2-(5-Méthoxy-2-phényl-3-benzo[b]furyl)éthyl]-3-butènamide

Point de fusion : 100°C
Microanalyse élémentaire :

| | C | H | N |
|---|---|---|---|
| % Calc | 75,20 | 6,31 | 4,18 |
| % Trouvé | 75,05 | 6,38 | 4,05 |

### Exemple 108 : N-[2-(5-Méthoxy-2-phényl-3-benzo[b]furyl)éthyl]-2-butènamide

Point de fusion: 119°C
Microanalyse élémentaire :

| | C | H | N |
|---|---|---|---|
| % Calc | 75,20 | 6,31 | 4,18 |
| % Trouvé | 75,13 | 6,34 | 4,25 |

### Exemple 109 : N-{2-[5-Méthoxy-2-(3-méthoxybenzyl)-3-benzo[b]furyl]éthyl} acrylamide

Le produit attendu est obtenu selon le procédé décrit dans l'exemple 1, en utilisant comme produit de départ le composé décrit dans la préparation D, et au stade b en remplaçant le chlorure d'acyle par le chlorure de l'acide acrylique.
Point de fusion: 114°C
Microanalyse élémentaire :

| | C | H | N |
|---|---|---|---|
| % Calc | 72,31 | 6,35 | 3,83 |
| % Trouvé | 72,69 | 6,40 | 3,81 |

### Exemple 110 : N-{2-[2-(3-Chlorobenzyl)-5-méthoxy-3-benzo[b]furyl]éthyl}acétamide

Le produit attendu est obtenu selon le procédé décrit dans l'exemple 1, en utilisant comme produit de départ le composé décrit dans la préparation AD.
Point de fusion : 115°C
Microanalyse élémentaire :

| | C | H | N | Cl |
|---|---|---|---|---|
| % Calc | 67,13 | 5,63 | 3,91 | 9,91 |
| % Trouvé | 67,02 | 5,70 | 3,85 | 9,75 |

### Exemple 111 : N-{2-[2-(3,5-Bistrifluorométhylbenzyl)-5-méthoxy-3-benzo[b]furyl] éthyl})acétamide

Le produit attendu est obtenu selon le procédé décrit dans l'exemple 1, en utilisant comme produit de départ le composé décrit dans la préparation AE.
Point de fusion : 125°C
Microanalyse élémentaire :

| | C | H | N | F |
|---|---|---|---|---|
| % Calc | 57,52 | 4,17 | 3,05 | 24,81 |
| % Trouvé | 57,27 | 4,09 | 3,08 | 24,75 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I] - iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs MEL₁ₐ et MEL_{1b} de la mélatonine

Les expériences de liaison aux récepteurs MEL₁ₐ ou MEL_{1b} sont réalisées en utilisant la 2-[¹²⁵I]-mélatonine comme radioligand de référence. La radioactivité retenue est déterminée à l'aide d'un compteur à scintillation liquide Beckman® LS 6000.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention montrent que la liaison des composés testés est très puissante pour l'un ou l'autre des sous-types de récepteurs MEL₁ₐ ou MEL_{1b}, ces valeurs se situant dans un intervalle de 0,1 à 10nM.

### EXEMPLE D : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE F : Activité Antiarythmique

### Protocole (Ref: LAWSON J.W.et al. J. Pharmacol. Expert. Therap., 1968, 160, pp 22-31)

La substance testée est administrée par voie intrapéritonéale à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE G : Composition pharmaceutique : Comprimés

| | |
|---|---|
| 1000 comprimés dosés à 5 mg du composé de l'exemple 4 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 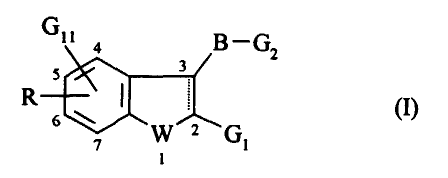 dans laquelle :
• la liaison 2-3 est saturée ou insaturée
• W représente un atome d'oxygène ou de soufre,
• B représente une chaîne alkylène (C₁-C₆) linéaire ou ramifiée éventuellement substituée par un ou plusieurs groupements alkyle(C₁-C₆) linéaire ou ramifié, hydroxy, alkoxy (C₁-C₆) linéaire ou ramifié, carboxy, et/ou alkoxycarbonyle (C₁-C₆) linéaire ou ramifié,
• R représente un atome d'hydrogène, un groupement hydroxy, un radical R' ou un groupement OR', R' représentant un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, trihalogénoalkylsulfonyl (C₁-C₆) linéaire ou ramifié, aryle éventuellement substitué, biphényle éventuellement substitué, ou hétéroaryle éventuellement substitué,
• G₁ représente un atome d'halogène, un groupement trihalogénoalkylsulfonyloxy (C₁-C₆) linéaire ou ramifié, carboxy, formyl, cyano, un radical R₁, un groupement -CO-R₁ ou -O-CO-R₁, R₁ représentant un groupement alkyle (C₁-C₆) linéaire ou ramifié éventuellement substitué, alkényle (C₂-C₆) linéaire ou rangé éventuellement substitué, alkynyle (C₂-C₆) linéaire ou ramifié éventuellement substitué, cycloalkyle (C₃-C₇) éventuellement substitué, aryle éventuellement substitué, biphényle éventuellement substitué, ou hétéroaryle éventuellement substitué,
et dans ce cas G₁₁ représente un atome d'hydrogène ou G₁₁ et R forment ensemble avec les atomes de carbone qui les portent un cycle de 5 à 7 chaînons, saturé ou insaturé, contenant un atome d'oxygène, ce cycle étant éventuellement substitué par un ou plusieurs groupements choisis parmi alkyle (C₁-C₆) linéaire ou ramifié, alkoxy (C₁-C₆) linéaire ou ramifié, hydroxy, oxo, carboxy, ou alkoxycarbonyle (C₁-C₆) linéaire ou ramifié,
ou bien,
G₁ représente un atome d'hydrogène, et dans ce cas, G₁₁ représente un groupement trihalogénoalkylsulfonyloxy (C₁-C₆) linéaire ou ramifié, carboxy, formyl, cyano, un radical R₁, un groupement -CO-R₁ ou -O-CO-R₁, R₁ représentant un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, aryle éventuellement substitué, biphényle éventuellement substitué, ou hétéroaryle éventuellement substitué,
• G₂ représente un groupement choisi parmi : 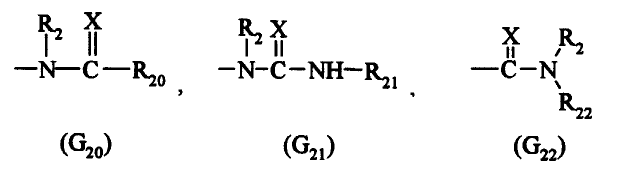 dans lesquels :
- X représente un atome d'oxygène ou de soufre,
- R₂ représente un atome d'hydrogène ou un groupement alkyle (C₁-C₆) linéaire ou ramifié,
- R₂₀, R₂₁, R₂₂ représentent un groupement alkyle (C₁-C₆) linéaire ou ramifié éventuellement substitué, alkényle (C₂-C₆) linéaire ou ramifié éventuellement substitué, alkynyle (C₂-C₆) linéaire ou ramifié éventuellement substitué, cycloalkyle (C₃-C₇) éventuellement substitué, hétéroaryle éventuellement substitué, aryle éventuellement substitué, ou biphényle éventuellement substitué,
étant entendu que :
- lorsque G₂ représente un groupement G₂₂ dans lequel R₂₂ tel que défini précédemment est différent d'un groupement aryle éventuellement substitué, hétéroaryle éventuellement substitué, ou biphényle éventuellement substitué, alors G₁ ou G₁₁ représentent un groupement hétéroaryle (différent de pyridyle) éventuellement substitué, un groupement alkyle (C₁-C₆) linéaire ou ramifié substitué par un groupement hétéroaryle (différent de pyridyle) éventuellement substitué, ou un groupement -CO-R₁ ou O-CO-R₁, R₁ étant tel que défini précédemment,
- lorsque G₂ représente un groupement G₂₀ ou G₂₁ dans lesquels R₂₀ et R₂₁ sont différents d'un groupement aryle éventuellement substitué, biphényle éventuellement substitué, ou hétéroaryle éventuellement substitué, et G₁ est différent d'un groupement hétéroaryle éventuellement substitué, ou naphtyle éventuellement substitué, ou G₁₁ est différent d'un groupement hétéroaryle éventuellement substitué, ou naphtyle éventuellement substitué tandis que R représente un atome d'hydrogène, alors B représente une chaîne méthylène ou éthylène éventuellement substituée,
- lorsque G₂ représente un groupement G₂₀ ou G₂₁, R₂₀ ou R₂₁ tels que définis précédemment étant différents d'un groupement aryle éventuellement substitué et d'un groupement hétéroaryle éventuellement substitué, et :
↪ lorsque R et G₁ représentent chacun un atome d'hydrogène, alors G₁₁ est différent d'un groupement alkyle (C₁-C₆) linéaire ou ramifié, d'un groupement cycloalkyle (C₃-C₇) éventuellement substitué par un ou plusieurs atomes d'halogène ou groupements hydroxy, et différent d'un groupement alkyle (C₁-C₆) linéaire ou ramifié substitué par un ou plusieurs atomes d'halogène ou groupements, hydroxy ou alkoxy (C₁-C₆) linéaire ou ramifié, ou cycloalkyle (C₃-C₇) éventuellement substitué par un ou plusieurs atomes d'halogène ou groupements hydroxy,
↪ lorsque G₁ représente un atome d'hydrogène et R représente un groupement OR', R' tel que défini précédemment étant différent d'un groupement hétéroaryle éventuellement substitué, alors G₁₁ est différent d'un groupement alkyle (C₁-C₆) linéaire ou ramifié, d'un groupement alkényle (C₂-C₆) linéaire ou ramifié, et d'un groupement alkynyle (C₂-C₆) linéaire ou ramifié
- lorsque G₂ représente soit un groupement G₂₁ dans lequel R₂₁ est différent d'un groupement alkényle (C₂-C₆) linéaire ou ramifié éventuellement substitué et d'un groupement alkynyle (C₂-C₆) éventuellement substitué, soit un groupement G₂₀ dans lequel R₂₀ représente un groupement cycloalkyle (C₃-C₇) éventuellement substitué par un ou plusieurs atomes d'halogène ou groupements hydroxy ou alkoxy (C₁-C₆) linéaire ou ramifié, ou un groupement alkyle (C₁-C₆) linéaire ou ramifié substitué par un groupement cycloalkyle (C₃-C₇) éventuellement substitué par un ou plusieurs atomes d'halogène ou groupements hydroxy ou alkoxy (C₁-C₆) linéaire ou ramifié, alors :
↪ G₁₁ est différent d'un groupement alkyle (C₁-C₆) linéaire ou ramifié,
↪ G₁ est différent d'un atome d'halogène, d'un groupement alkyl (C₁-C₆) linéaire ou ramifié, d'un groupement alkyle (C₁-C₆) linéaire ou ramifié substitué par un groupement phényle éventuellement substitué, et d'un groupement phényle éventuellement substitué,
- lorsque G₂ représente un groupement G₂₁, X représentant un atome de soufre et B une chaîne éthylène, alors R₂₁ est différent d'un groupement aryle éventuellement substitué,
le terme aryle désignant un groupement phényle ou naphtyle,
le terme hétéroaryle désignant un groupement mono ou bicyclique de 4 à 11 chaînons saturé ou insaturé contenant de 1 à 4 hétéroatomes choisis parmi azote, oxygène et soufre,
étant entendu que :
- le terme éventuellement substitué affectant les expressions alkyle, alkényle, alkynyle, cycloalkyle, signifie que ces groupements peuvent être substitués par un ou plusieurs atomes d'halogène, ou groupements cycloalkyle (C₃-C₇), hydroxy, alkoxy (C₁-C₆) linéaire ou ramifié, aryle éventuellement substitué, hétéroaryle éventuellement substitué,
- le terme éventuellement substitué affectant les expressions aryle, biphényle, et hétéroaryle signifie que ces groupements peuvent être substitués par un ou plusieurs atomes d'halogène, ou groupements alkyle (C₁-C₆) linéaire ou ramifié, trihalogénoalkyle (C₁-6₆) linéaire ou ramifiés, hydroxy, alkoxy (C₁-C₆) linéaire ou ramifié, nitro, amino (éventuellement substitué par un ou deux groupements alkyle (C₁-C₆) linéaire ou ramifié identiques ou différents), alkylcarbonyle (C₁-C₆) linéaire ou ramifié, cyano, carboxy, ou aminocarbonyle (éventuellement substitué par un ou deux groupements alkyle (C₁-C₆) linéaires ou ramifié, identiques ou différents),
leurs énantiomères, diastéréoisomères, ainsi que leurs sels d'addition à un acide ou à une base pharmaceutiquement acceptable.

2. Composés de formule (I) selon la revendication 1 tels que la liaison 2-3 est insaturée, leurs énantiomères, diastéréoisomères, ainsi que leurs sels d'addition à un acide ou à une base pharmaceutiquement acceptable.

3. Composés de formule (I) selon la revendication 1 tels que W représente un atome d'oxygène, leurs énantiomères, diastéréoisomères, ainsi que leurs sels d'addition à un acide ou à une base pharmaceutiquement acceptable.

4. Composés de formule (I) selon la revendication 1 tels que W représente un atome de soufre, leurs énantiomères, diastéréoisomères, ainsi que leurs sels d'addition à un acide ou à une base pharmaceutiquement acceptable.

5. Composés de formule (I) selon la revendication 1 tels que B représente un chaîne alkylène (C₁-C₆) linéaire ou ramifié, leurs énantiomères, diastéréoisomères, ainsi que leurs sels d'addition à un acide ou à une base pharmaceutiquement acceptable.

6. Composés de formule (I) selon la revendication 1 tels que G₁₁ représente un atome d'hydrogène, leurs énantiomères, diastéréoisomères, ainsi que leurs sels d'addition à un acide ou à une base pharmaceutiquement acceptable.

7. Composés de formule (I) selon la revendication 1 tels que G₁ et R représentent chacun un atome d'hydrogène, leurs énsantiomères, diastéréoisomères, ainsi que leurs sels d'addition à un acide ou à une base pharmaceutiquement acceptable.

8. Composés de formule (I) selon la revendication 1 tels que R est rattaché à la position 5 du noyau hétérocyclique, leurs énantiomères, diastéréoisomères, ainsi que leurs sels d'addition à un acide ou à une base pharmaceutiquement acceptable.

9. Composés de formule (I) selon la revendication 1 tels que G₁₁ rattaché à la position 5 du noyau hétérocyclique, leurs énantiomères, diastéréoisomères, ainsi que leurs sels d'addition à un acide ou à une base pharmaceutiquement acceptable.

10. Composés de formule (I) selon l'une quelconque des revendications 1 ou 8 tels que R représente un atome d'hydrogène ou un radical R' ou OR' dans lesquels R' représente un groupement alkyle (C₁-C₆) linéaire ou ramifié, alkényle (C₂-C₆) linéaire ou ramifié ou alkynyle (C₂-C₆) linéaire ou ramifié, leurs énantiomères, diastéréoisomères, ainsi que leurs sels d'addition à un acide ou à une base pharmaceutiquement acceptable.

11. Composés de formule (I) selon la revendication 1 tels que G₁ représente un groupement aryle éventuellement substitué, hétéroaryle éventuellement substitué, ou un groupement alkyle (C₁-C₆) linéaire ou ramifié substitué par un groupement choisi parmi cycloalkyle (C₃-C₇), aryle éventuellement substitué, et hétéroaryle éventuellement substitué, leurs énantiomères, diastéréoisomères, ainsi que leurs sels d'addition à un acide ou à une base pharmaceutiquement acceptable.

12. Composés de formule (I) selon l'une quelconque des revendications 1 ou 11 tels que G₁ représente un groupement méthylène substitué par un groupement phényle éventuellement substitué, leurs énantiomères, diastéréoisomères, ainsi que leurs sels d'addition à un acide ou à une base pharmaceutiquement acceptable.

13. Composés de formule (I) selon l'une quelconque des revendications 1 ou 9 tels que G₁₁ représente un groupement aryle éventuellement substitué, hétéroaryle éventuellement substitué, ou un groupement alkyle (C₁-C₆) linéaire ou ramifié substitué par un groupement choisi parmi cycloalkyle (C₃-C₇), aryle éventuellement substitué, et hétéroaryle éventuellement substitué, leurs énantiomères, diastéréoisomères, ainsi que leurs sels d'addition à un acide ou à une base pharmaceutiquement acceptable.

14. Composés de formule (I) selon la revendication 1 pour lesquels G₂ est tel que X représente un atome d'oxygène, R₂ représente un atome d'hydrogène et R₂₀, R₂₁ et R₂₂ sont choisis parmi les groupements alkényle (C₂-C₆) linéaire ou ramifié, et alkyle (C₁-C₆) linéaire ou ramifié éventuellement substitué par un ou plusieurs atomes d'halogène, leurs énantiomères, diastéréoisomères, ainsi que leurs sels d'addition à un acide ou à une base pharmaceutiquement acceptable.

15. Composés de formule (I) selon l'une quelconque des revendications 1 à 14 tels que la liaison 2-3 est insaturée, W représente un atome d'oxygène, B représente une chaîne éthylène, R, rattaché en position 5 du noyau hétérocyclique représente un atome d'hydrogène ou un groupement R' ou OR' dans lesquels R' est un groupement alkyle (C₁-C₆) linéaire ou ramifié, G₁₁ représente un atome d'hydrogène, G₁ représente un groupement aryle éventuellement substitué, hétéroaryle éventuellement substitué, ou un groupement alkyle (C₁-C₆) linéaire ou ramifié substitué par un groupement cycloalkyle (C₃-C₇), aryle éventuellement substitué, ou par un groupement hétéroaryle éventuellement substitué , et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par un ou plusieurs atomes d'halogène, leurs énantiomères, diastéréoisomères, ainsi que leurs sels d'addition à un acide ou à une base pharmaceutiquement acceptable.

16. Composés de formule (I) selon l'une quelconque des revendications 1 à 14 tels que la liaison 2-3 est insaturée, W représente un atome d'oxygène, B représente une chaîne éthylène, R et G₁ représentent chacun un atome d'hydrogène, G₁₁ rattaché en position 5 du noyau hétérocyclique représente un groupement aryle éventuellement substitué, hétéroaryle éventuellement substitué, ou un groupement alkyle (C₁-C₆) linéaire ou ramifié substitué, par un groupement aryle éventuellement substitué ou hétéroaryle éventuellement substitué, et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par un ou plusieurs atomes d'halogène, leurs énantiomères, diastéréoisomères, ainsi que leurs sels d'addition à un acide ou à une base pharmaceutiquement acceptable.

17. Composés de formule (I) selon l'une quelconque des revendications 1 à 14 tels que la liaison 2-3 est insaturée, W représente un atome de soufre, B représente une chaîne éthylène, R, rattaché en position 5 du noyau hétérocyclique représente un atome d'hydrogène ou un groupement R' ou OR' dans lesquels R' est un groupement alkyle (C₁-C₆) linéaire ou ramifié, G₁₁ représente un atome d'hydrogène, G₁ représente un groupement aryle éventuellement substitué, hétéroaryle éventuellement substitué, ou un groupement alkyle (C₁-C₆) linéaire ou ramifié substitué par un groupement cycloalkyle (C₃-C₇), aryle éventuellement substitué, ou par un groupement hétéroaryle éventuellement substitué, et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par un ou plusieurs atomes d'halogène, leurs énantiomères, diastéréoisomères, ainsi que leurs sels d'addition à un acide ou à une base pharmaceutiquement acceptable.

18. Composés de formule (I) selon l'une quelconque des revendications 1 à 14 tels que la liaison 2-3 est insaturée, W représente un atome de soufre, B représente une chaîne éthylène, R et G₁ représentent chacun un atome d'hydrogène, G₁₁ rattaché en position 5 du noyau hétérocyclique représente un groupement aryle éventuellement substitué, hétéroaryle éventuellement substitué, ou un groupement alkyle (C₁-C₆) linéaire ou ramifié substitué, par un groupement aryle éventuellement substitué ou hétéroaryle éventuellement substitué, et G₂ représente un groupement G₂₀ dans lequel X représente un atome d'oxygène, R₂ représente un atome d'hydrogène et R₂₀ représente un groupement alkényle (C₂-C₆) linéaire ou ramifié, ou un groupement alkyle (C₁-C₆) linéaire ou ramifié éventuellement substitué par un ou plusieurs atomes d'halogène, leurs énantiomères, diastéréoisomères, ainsi que leurs sels d'addition à un acide ou à une base pharmaceutiquement acceptable.

19. Composé de formule (I) selon la revendication 1 qui est le N-[2-(2-benzyl-5-méthoxy-3-benzo[*b*]furyl)éthyl]acrylamide.

20. Composés de formule (I) selon la revendication 1 qui sont les :
- N-{2-[5-méthoxy-2-(3-méthoxybenzyl)-3-benzo[*b*]furyl]éthyl}acétamide
- N-{2-[5-méthoxy-2-(3-trifluorométhylbenzyl)-3-benzo[*b*]furyl]éthyl}acétamide

21. Composé de formule (I) selon la revendication 1 qui est le N-{2-[5-(2-méthoxyphényl)-3-benzo[*b*]furyl]éthyl}acétamide.

22. Procédé de préparation des composés de formule (I) caractérisé en ce que l'on utilise comme matière première un dérivé de formule (II) : 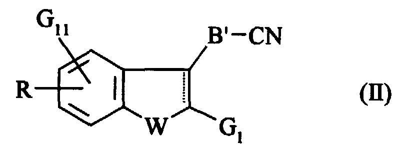 dans laquelle R, W, G₁ et G₁₁ sont tels que définis dans la formule (I), et B' représente une chaîne alkylène (C₀-C₅) linéaire ou ramifié éventuellement substitué par un ou plusieurs groupements alkyle(C₁-C₆) linéaire ou ramifié, hydroxy, alkoxy (C₁-C₆) linéaire ou ramifié, carboxy, et/ou alkoxycarbonyle (C₁-C₆) linéaire ou ramifié,
que l'on soumet :
↪ soit à une réaction de réduction qui peut être une hydrogénation catalytique ou une réduction chimique (par exemple en utilisant le complexe borane-tétrahydrofurane), pour conduire au composé de formule (III) : 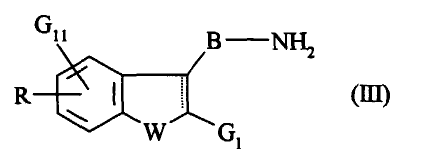 dans laquelle R, W, G₁ et G₁₁ sont tels que définis précédemment, et B est tel que défini dans la formule (I),
a) qui peut être soumis à l'action d'un chlorure d'acyle de formule (IV) 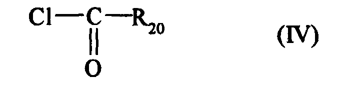 dans laquelle R₂₀ est tel que défini dans la formule (I),
pour conduire à un composé de formule (I/a) : 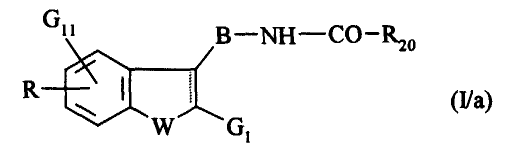 cas particulier des composés de formule (I) pour lequel R, W, B, G₁, G₁₁ et R₂₀ sont tels que définis précédemment,
composé I/a qui peut être, si on le désire, soumis à une réaction d'alkylation classique en utilisant comme réactif un composé de formule P-R'₂, dans laquelle P représente un groupe partant (tel qu'un halogène ou un groupement tosyle) et R'₂, différent d'un atome d'hydrogène, a la même signification que R₂ dans la formule (I), ou bien en utilisant un dialkylsulfate, pour conduire à un composé de formule (I/b) : 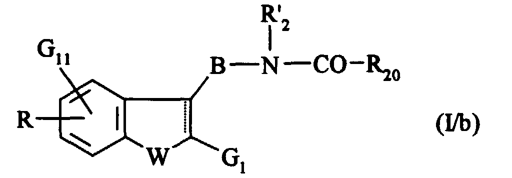 cas particulier des composés de formule (I) pour lequel R, W, B, G₁, G₁₁, R'₂ et R₂₀ sont tels que définis précédemment,
composés (I/a) et (I/b) qui peuvent être soumis à un agent de thionation, par exemple le réactif de Lawesson, pour obtenir un composé de formule (I/c) : 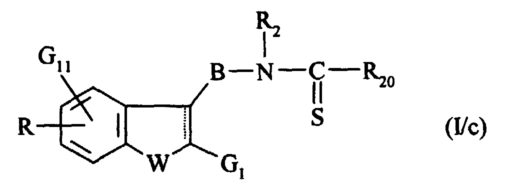 cas particulier des composés de formule (I) pour lequel R, W, B, G₁, G₁₁, R₂ et R₂₀ sont tels que définis précédemment,
b) ou bien qui peut être soumis à l'action d'un iso(thio)cyanate de formule (V) :
X=C=N-R₂₁ (V)
dans laquelle X et R₂₁ sont tels que définis dans la formule (I),
pour conduire à un composé de formule (I/d) : 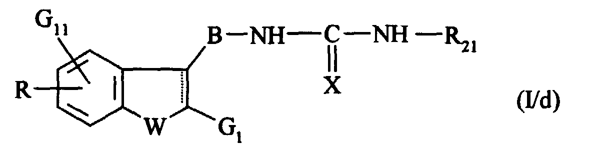 cas particulier des composés de formule (I) pour lequel R, W, B, G₁, G₁₁, X et R₂₁ sont tels que définis précédemment,
composé (I/d) qui peut être soumis à une réaction d'alkylation en utilisant un agent classique d'alkylation de formule P-R'₂, dans laquelle P est un groupe partant (par exemple un atome d'halogène ou un groupement tosyle) et R'₂, différent d'un atome d'hydrogène, a la même signification que R₂ dans la formule (I), ou bien en utilisant un dialkylsulfate, pour conduire à un composé de formule (I/e) : 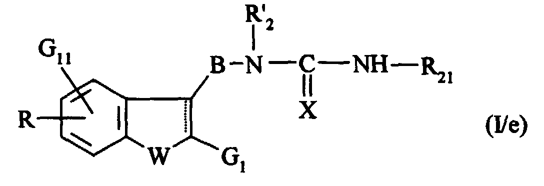 cas particulier des composés de formule (I) pour lequel R, W, B, G₁, G₁₁, R'₂, X et R₂₁ sont tels que définis précédemment,
↪ soit à une réaction d'hydrolyse en milieu acide ou basique pour conduire à l'acide carboxylique correspondant, de formule (VI) : 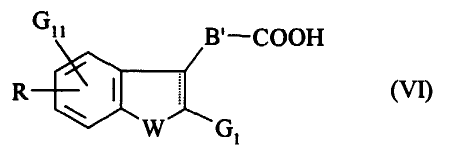 dans laquelle R, W, G₁ et G₁₁ ont la même signification que dans la formule (I), et B' est tel que défini précédemment,
que l'on fait réagir, après formation éventuellement du chlorure d'acide correspondant, avec un composé de formule (VII) : 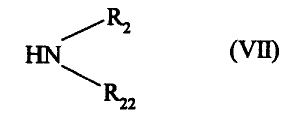 dans laquelle R₂ et R₂₂ sont tels que définis dans la formule (I),
pour conduire à un composé de formule (I/f) : 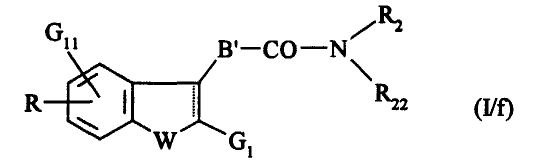 cas particulier des composés de formule (I) pour lequel R, W, B', G₁, G₁₁, R₂ et R₂₂ sont tels que définis précédemment,
composé de formule (I/f) qui peut, si on le désire, être soumis à un agent de thionation, par exemple le réactif de Lawesson, pour conduire à un composé de formule (I/g) : 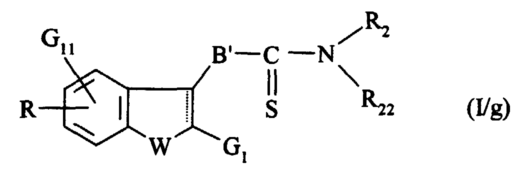 cas particulier des composés de formule (I) pour lequel R, W, B', G₁, G₁₁, R₂ et R₂₂ sont tels que définis précédemment,
composés de formule (I/a), (I/b), (I/c), (I/d), (I/e), (I/f), (I/g) qui lorsque R représente un groupement O-Alk (-Alk représentant un groupement alkyle (C₁-C₆) linéaire ou ramifié) et G₁₁ un groupement G'₁₁ ne pouvant former avec R un cycle, peuvent être, lorsque cela est compatible avec les substituants présents sur la molécule, traités par le tribromure de bore pour conduire au composé hydroxylé correspondant, de formule (VIII) : 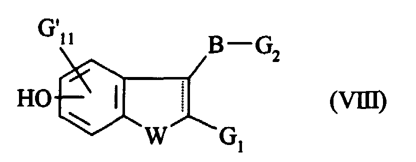 dans laquelle W, G₁, B et G₂ sont tels que définis dans la formule (I), et G'₁₁ a la même définition que G₁₁ dans la formule (I),
composé de formule (VIII), dont la fonction hydroxyle peut être :
a) soit transformée en trifluorométhanesulfonate, en utilisant par exemple le phényl bis(trifluorométhanesulfonimide) en milieu basique, pour conduire au composé de formule (IX) : 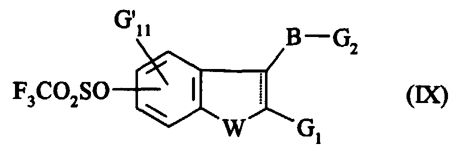 dans laquelle W, G₁, G'₁₁, B et G₂ sont tels que définis précédemment, que l'on peut transformer, par l'intermédiaire d'une réaction catalysée par un dérivé du palladium (O) en utilisant comme réactif un dérivé de l'acide borique (R'B(OH)₂) ou un dérivé de l'étain (R'SnBu₃), pour lesquels R' est tel que défini dans la formule (I), pour obtenir un composé de formule (I/h) : 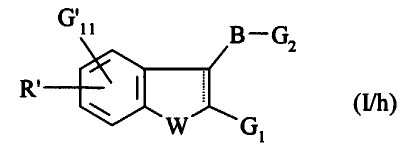 cas particulier des composés de formule (I) pour lequel R', W, G₁, G'₁₁, B et G₂ sont tels que définis précédemment,
b) soit soumise à une réaction de O-substitution, en milieu basique, en utilisant comme réactif le dérivé halogéné approprié, pour conduire à un composé de formule (I/i) : 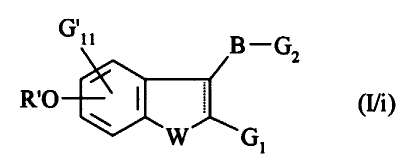 cas particulier des composés de formule (I) pour lequel R', W, G₁, G'₁₁, B et G₂ sont tels que définis précédemment,
c) soit, lorsque G'₁₁ représente un atome d'hydrogène, soumise à une réaction de O-substitution pour conduire à un composé de formule (X) : 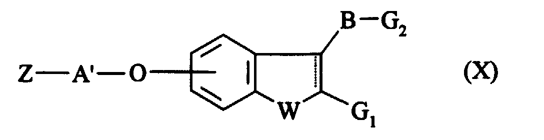 dans laquelle W, G₁, G₂ et B sont tels que définis précédemment, A' représente une chaîne alkylène (C₁-C₃), alkénylène (C₂-C₃) ou alkylène (C₂-C₃), et Z représente une fonction réactive (par exemple un acide carboxylique ou un ester) ou bien forme avec A' une liason double ou triple,
qui peut être cyclisé, par l'intermédiaire d'une réaction de substitution électrophile intra-moléculaire lorsque Z représente une fonction réactive ou par l'intermédiaire d'un réarrangement de claisen lorsque Z forme avec A' une liaison double ou triple, pour conduire à un composé de formule (I/k) : 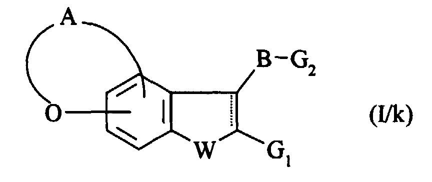 cas particulier des composés de formule (I) pour lequel W, G₁, G₂, et B sont tels que définis précédemment, et A représente une chaîne alkylène (C₂-C₄), alkénylène (C₃-C₄), alkynylène (C₃-C₄), ou une chaîne alkylène (C₂-C₄) substituée par un groupement oxo, ce dernier pouvant être, si on le désire, transformé en un groupement hydroxy, alkoxy (C₁-C₆) linéaire ou ramifié, alkyle (C₁-C₆) linéaire ou ramifié,
composés (I/a) à (I/k) et (IX) formant la totalité des composés de formule (I),
- que l'on purifie éventuellement selon une technique classique de purification,
- dont on sépare éventuellement les énantiomères selon une technique classique de séparation,
- et que l'on transforme, le cas échéant, en leurs sels d'addition à un acide ou à une base pharmaceutiquement acceptable

23. Compositions pharmaceutiques contenant comme principe actif au moins un composé selon l'une quelconque des revendications 1 à 21, seul ou en combinaison avec un ou plusieurs excipients ou véhicules inertes, non toxiques, pharmaceutiquement acceptables.

24. Compositions pharmaceutiques selon la revendication 23 contenant au moins un principe actif selon l'une quelconque des revendications 1 à 21 utiles pour le traitement des troubles liés au système mélatoninergique.
